**Europäisches Patentamt**

**European Patent Office**

**Office européen des brevets**

(19)

(11) Numéro de publication: **0 198 745 B1**

(12)

# FASCICULE DE BREVET EUROPEEN

(45) Date de publication du fascicule du brevet:
**16.05.90**

(21) Numéro de dépôt: **86400617.6**

(22) Date de dépôt: **24.03.86**

(51) Int. Cl.⁵: **C12N 15/00,** C12P 21/02,
A61K 37/02
// (C12P21/02, C12R1:19)

(54) **Procédé de préparation microbiologique de la sérum-albumine humaine.**

(30) Priorité: **25.03.85 FR 8504384**

(43) Date de publication de la demande:
**22.10.86 Bulletin 86/43**

(45) Mention de la délivrance du brevet:
**16.05.90 Bulletin 90/20**

(84) Etats contractants désignés:
**AT BE CH DE FR GB IT LI LU NL SE**

(56) Documents cités:
**EP-A- 0 073 646
EP-A- 0 079 739
EP-A- 0 091 527
EP-A- 0 114 506
EP-A- 0 138 437**

**PROCEEDINGS OF THE NATIONAL ACADEMY OF
SCIENCES OF THE USA,
vol. 81, no. 3, février 1984, pages 669-673, Washington,
US; M. COURTNEY et al.: "High-level production of
biologically active human alpha1-antitrypsin in
Escherichia coli"**

(73) Titulaire: **GENETICA, 160 Quai de Polangis,
94340 Joinville Le Pont(FR)**

(72) Inventeur: **Knapp, Michael, 17 Place des Vosges,
F-75004 Paris(FR)**
Inventeur: **Brefort, Georges, 3 rue Las Cases,
F-75007 Paris(FR)**
Inventeur: **Sarmientos, Paolo, 73 rue de Wattignies,
F-75812 Paris(FR)**

(74) Mandataire: **Pilard, Jacques et al, RHONE-POULENC
RECHERCHES Service Brevets Pharma 25, Quai Paul
Doumer, F-92408 Courbevoie Cedex(FR)**

EP 0 198 745 B1

## Description

La présente invention concerne un nouveau procédé de synthèse protéique.

Plus particulièrement, l'invention concerne un procédé mettant en oeuvre les techniques de manipulations génétiques *in vitro*, pour obtenir un réarrangement concerté de séquences d'acide déoxyribonucléique permettant d'induire la synthèse de la sérum-albumine humaine par une bactérie.

La sérum-albumine humaine est une protéine constituée de 585 acides aminés, ne contenant pas de résidus glycosidiques associés et ayant un poids moléculaire de l'ordre de 66000 daltons.

Génétiquement, la sérum-albumine humaine est codée chez l'homme par deux gènes alléliques autosomiques et codominants. Les gènes de la sérum-albumine humaine sont notoirement polymorphiques, et l'on connaît au moins vingt-quatre variants de sérum-albumine différenciés par leur comportement électrophorétique (Shell et Blumberg, "The genetics of Human serum-albumin", dans "Albumin Structure, Function and Uses", Rosenoer, Oratz et Rothschild éds., Pergamon Press, 1977).

La sérum-albumine est synthétisée dans les hépatocytes, puis excrétée dans le sérum dont elle constitue la protéine la plus abondante, avec des concentrations moyennes de l'ordre de 4 g/100 ml de sérum. Elle joue un rôle physiologique majeur dans le maintien de la pression osmotique du plasma, et contribue ainsi à la stabilité de l'équilibre entre milieu intérieur (cellulaire) et milieu extérieur (circulant), équilibre qui assure, entre autres fonctions, le maintien d'un taux d'hydratation cellulaire compatible avec le fonctionnement physiologique normal de l'organisme.

La sérum-albumine humaine joue également un rôle dans le transport de molécules hydrophobes "naturelles" (stéroïdes et sels biliaires par exemple) ou médicamenteuses jusqu'à leurs sites d'action.

Ceci explique que la sérum-albumine humaine soit utilisée soit dans la thérapeutique des troubles de la volémie, par exemple hypovolémies aigües post-hémorragiques, brûlures étendues, soit en thérapeutique d'appoint dans les solutés dits de remplissage en chirurgie générale, soit dans le traitement des états de déshydratation (par exemple des syndromes néphrotiques), toutes ces utilisations exigeant l'apport de quantités considérables de sérum-albumine (plusieurs dizaines de grammes par jour par malade).

La sérum-albumine humaine est extraite actuellement du sérum par des techniques dérivées de celle de E.J. Cohn et coll., J. Am. Chem. Soc. (1946), 68, p. 459 et suivantes, ou de placentas par la technique de J. Liautaud et coll., 13th Internat. Congress of IABS, Budapest ; A : Purification of Proteins. Development of Biological Standard (1973) Karger, éd., Bale, 27, p. 107 et suivantes. Ces sources, qui suffisent à peine aux besoins du marché mondial, souffrent de plusieurs défauts, entre autres leur caractère aléatoire. Par ailleurs, elles ne sont pas dépourvues de risques de contaminations (hépatite par exemple, et plus récemment syndrome immuno-déficitaire acquis), ce qui aurait des conséquences dramatiques lors d'une utilisation thérapeutique.

Les techniques de recombinaison génétique *in vitro* offrent maintenant la possibilité de faire synthétiser par un microorganisme, par exemple la bactérie Escherichia coli, n'importe quelle protéine ou n'importe quel polypeptide, et ce théoriquement en quantités illimitées (voir par exemple F. Gros et coll., Sciences de la Vie et Société, Documentation Française éd. 1979).

On sait, depuis les expériences classiques de F. Jacob et coll., que l'ADN contient d'une part un ensemble de gènes dits "de structure", c'est-à-dire codant pour une protéine donnée, et d'autre part des gènes dits "de régulation", c'est-à-dire capables de moduler l'expression des gènes de structure, l'association des deux types formant une entité dite "opéron".

Les recherches en biologie moléculaire et la mise au point des techniques de séquençage de l'ADN (F. Sanger et A.R. Coulson, J. Mol. Biol. (1975), 94, p. 441 et suivantes ; A.M. Maxam et W. Gilbert, Proc. Natl. Acad. Sci. (USA) (1977), 74, p. 560 et suivantes) ont permis de préciser l'organisation de l'opéron telle que l'avaient conçue F. Jacob et J. Monod (F. Jacob et J. Monod, Cold Spring Harbor Symp. Quant. Biol. (1961), 26, p. 193 et suivantes ; F. Jacob et J. Monod, J. Mol. Biol. (1961), 3, p. 318 et suivantes), et d'identifier les caractères particuliers de la structure primaire des deux types de gènes.

Ainsi, tout gène de structure est encadré d'un codon dit "d'initiation de traduction" (ATG) et d'un codon "d'arrêt". Le codon d'initiation a pour fonction de fixer un ARN de transfert porteur d'une formylméthionine. La chaîne protéique s'allongera à partir de cette formylméthionine par attachements successifs des acides aminés codés par le gène de structure ; le codon "d'arrêt" provoquera enfin un arrêt de l'allongement et la libération de la protéine néoformée.

En ce qui concerne les gènes régulateurs (promoteurs, répresseurs), définissant par exemple un promoteur comme un fragment d'ADN sur lequel se fixe l'ARN polymérase, on a pu identifier les séquences les plus conservées (D. Pribnow, Proc. Natl. Acad. Sci. (USA) (1975), 72, p. 784 et suivantes) ; de même on a pu définir les séquences d'ADN les plus conservées au niveau des sites de fixation des ribosomes (RBS) (J. Shine et L. Dalgarno, Nature (1975), 254, p. 34 et suivantes), sites qui jouent un rôle dans la traduction en protéine de l'ARN transcrit.

Ainsi les gènes régulateurs bactériens peuvent donc être définis par leurs propriétés fonctionnelles et aussi par leur séquence primaire, ce dont les techniques de recombinaisons génétiques *in vitro* tirent profit pour placer un gène de structure quelconque sous leur contrôle, ceci grâce à l'existence des "enzymes de restriction", qui coupent l'ADN en des points spécifiques (H.O. Smith et K.W. Wilcox, J. Mol. Biol. (1970), 51, p. 379 et suivantes ; M. Meselson et R. Yuan, Nature (1968), 217, p. 1110 et suivantes ; R.J. Roberts, Nucleic Acids Res. (1982), 1, p. 135 et suivantes).

2

Les techniques utilisées, et connues par ailleurs, mettent en oeuvre l'utilisation concertée de ces enzymes pour couper l'ADN en des points prédéterminés, et d'enzymes dites "ligases" pour lier les fragments entre eux (P.E. Loban et A.D. Kaiser, J. Mol. Biol. (1973), 78, p. 453 et suivantes). L'ensemble est porté par des "vecteurs" (plasmides ou bactériophages), susceptibles d'être introduits dans une bactérie telle que E.coli selon des procédés connus par ailleurs, et de s'y maintenir lors de la croissance de la bactérie-hôte (M. Mandel et A. Higa, J. Mol. Biol. (1970), 53, p. 154 et suivantes).

Ainsi la présente invention concerne un procédé permettant d'induire la biosynthèse de la sérum-albumine humaine dans un microorganisme.

L'invention consiste à modifier in vitro le gène de structure de la sérum-albumine humaine de telle sorte qu'il possède un codon d'initiation, puis à lier le gène de structure modifié à un gène régulateur inductible.

Une bactérie-hôte, telle que E.coli, contenant le gène modifié, produit un taux d'albumine important après induction dans des conditions définies.

R.G. Schoner et coll., Biotechnology (1985), p. 151 et suivantes ont observé que des protéines hétérologues synthétisées à haut niveau dans E.coli se trouvent dans la cellule sous forme d'aggrégats insolubles. Ce même phénomène est observé dans le cas de la sérum-albumine humaine synthétisée par E.coli. L'insolubilité de la sérum-albumine humaine produite par E.coli peut être expliquée, par exemple, par le fait que la protéine pourrait ne pas être synthétisée dans sa conformation native en particulier parce que le potentiel d'oxydo-réduction intracellulaire est incompatible avec la formation correcte des 17 ponts disulfures de la protéine (J. Uren, International Biotechnology Laboratory, Avril 1985, p. 26 et suivantes). Il a été montré, dans d'autres systèmes, que des protéines mal repliées ont tendance à s'aggréger et à précipiter (J. London et coll., Eur. J. Biochem (1974), 47, p. 409 et suivantes ; G. Orsini et M.E. Goldberg, J. Biol. Chem. (1978), 253, p. 3453 et suivantes). Il en résulte que la sérum-albumine humaine synthétisée chez E.coli ne peut pas être purifiée par des méthodes classiques et n'est pas un produit directement utilisable en thérapeutique.

De nombreuses protéines peuvent être transformées en leur forme native après dénaturation et réduction contrôlées in vitro. Il en est ainsi par exemple, pour la tryptophanase d'E.coli, le chymotrypsinogène, l'anhydrase carbonique, la ribonucléase, le lysozyme et l'inhibiteur trypsique de pancréas bovin (J. London et coll., Eur. J. Biochem. (1974), 47, p. 409 et suivantes ; G. Orsini et M.E. Goldberg, J. Biol. Chem. (1978), 253, p. 3453 et suivantes ; R.F. Goldberg et coll., J. Biol. Chem. (1970), 9, p. 5015 et suivantes ; T.E. Creighton, J. Mol. Biol. (1974), 87, p. 563 et suivantes). Dans la plupart des cas, l'indicateur le plus sensible de renaturation complète d'une protéine est son activité enzymatique. Cependant une activité enzymatique n'a pas pu être mise en évidence de façon indiscutable pour la sérum-albumine. La renaturation de la sérum-albumine bovine (J.M. Teale et D.G. Benjamin, J. Biol. Chem. (1976), 251, p. 4603 et suivantes) et humaine (A. Wichman et coll., Eur. J. Biochem. (1977), 79 p. 339 et suivantes) a été possible à l'aide de techniques immunologiques en utilisant des anticorps polyclonaux. Plus récemment, il a été montré que certains anticorps monoclonaux peuvent servir de sondes de la conformation native d'une protéine (L. Djavadi - Ohaniance et coll., Biochemistry (1984), 23, p. 97 et suivantes). Il est donc possible d'analyser la conformation de la sérum-albumine humaine à l'aide d'anticorps monoclonaux dirigés contre cette protéine et qui sont décrits dans la littérature (C. Lapresle et N. Doyen, Mol. Immunol. (1983), 20, p. 549 et suivantes).

La présente invention concerne également un procédé permettant de modifier, par dénaturation et renaturation contrôlée, la conformation de la sérum-albumine humaine, obtenue par voie microbiologique selon le procédé de la présente invention ou par toute autre méthode conduisant à une sérum-albumine humaine non-native, afin que la protéine ainsi produite possède une structure tridimensionnelle identique à celle de la sérum-albumine humaine native d'origine naturelle.

Dans ce qui suit la signification des termes techniques employés en Biologie Moléculaire sera supposée connue (cf. par exemple "Biologie Moléculaire du Gène", de J. Watson, édition française, Interéditions 1978). Dans ce qui suit seront décrits successivement la construction, les procédés d'expression du gène et la renaturation de la sérum-albumine humaine.

## A. CONSTRUCTION DU GÈNE DE LA SERUM-ALBUMINE HUMAINE

### 1. Préparation d'ARN messager de foie

On utilise des cellules hépatiques, obtenues par exemple par biopsie, et on en extrait l'ARN messager selon la méthode décrite par exemple par V. Glisin et coll., Biochemistry (1974), 13, p. 2633 et suivantes ; et par R. Deeley et coll., J. Biol. Chem. (1977), 252, p. 8310 et suivantes. On traite la biopsie par une solution de thiocyanate de guanidine 6M, et l'on purifie l'ARN total par plusieurs cycles de précipitation dans l'éthanol à -20°C, centrifugation et redissolution des culots de centrifugation.

On enrichit la préparation en ARN messager par plusieurs cycles de chromatographie d'affinité sur des colonnes d'oligo (dT)-cellulose, selon la technique décrite par H. Aviv et P. Leder, Proc. Natl. Acad. Sci. (USA) (1972), 69, p. 1408 et suivantes. L'ARN messager ainsi isolé, contenant 1 à 2 % de l'ARN total, est conservé en solution aqueuse à -70°C.

On peut déterminer la proportion d'ARN messager spécifique de la sérum-albumine humaine au sein de la population totale (par exemple par traduction *in vitro* d'un aliquot de la solution d'ARN dans des lysats de réticulocytes de lapin). Une méthode consiste à utiliser le lysat de réticulocytes fournis par la société Amersham, suivant le protocole préconisé par ce fournisseur. On peut ainsi déterminer la fraction de protéine néoformée immunoprécipitable par des anticorps anti-albumine au sein de l'ensemble des protéines néoformées. On obtient par exemple une fraction de l'ordre de 6 %.

### 2. Synthèse de cDNA et clonage dans E.coli

#### a. Synthèse du premier brin

A partir de la technique de G.N. Buell et coll., J. Biol. Chem. (1978), 253, p. 2471 et suivantes, modifiée, on utilise par exemple 5 µg d'ARN messager total dans un volume final de 50 microlitres d'une solution contenant : 100 mM Tris-HCl pH 8.3, 10 mM MgCl$_2$, 0,4 mM DTT, 20 mM KCl, 0,4mM Na pyrophosphate, 1mM de chaque nucléotide triphosphate (dNTP) ,100 µg/ml de oligo(dT)$_{12-18}$, 0,5 U/ml d'inhibiteur de ribonucléases, 50 picomoles de traceur radioactif et 40 unités de Transcriptase réverse (Société Life Science, Inc.).

La réaction de transcription réverse de l'ARN messager en ADN complémentaire (cDNA) se poursuit pendant 1 heure à 42°C.

Le taux de synthèse de cDNA est calculé par mesure du taux d'incorporation du traceur radioactif en molécules acido-précipitables, selon une technique connue.

Après 1 heure, on arrête la réaction par addition d'EDTA (20 mM), et l'on détruit l'ARN messager par digestion alcaline dans 50 mM de NaOH, à 42°C, pendant 3 heures.

On sépare le cDNA néoformé des dNTPs non-incorporés et des produits de dégradation alcaline des ARNs par chromatographie, par exemple, sur une colonne de Sephadex G100 (Pharmacia Fine Chemicals). On obtient 1,5 µg de cDNA simple brin à partir de 5 µg d'ARN messager total.

#### b. Synthèse du deuxième brin

Le cDNA simple brin est converti en ADN double brin par action du fragment "Klenow" de l'ADN polymérase I.

Les conditions de réaction sont : 100 mM Hepes pH 7, 10 mM MgCl$_2$, 2,5 mM DTT, 70 mM KCl, 0,5 mM de chaque dNTP, et 50 unités du fragment "Klenow" de l'ADN polymérase I (commercialisée par exemple par la Société New England Biolabs Inc.).

La réaction est poursuivie pendant 15 heures, à 15°C, et l'on sépare l'ADN double brin des dNTPs non incorporés à nouveau par chromatographie sur colonne de Sephadex G100.

#### c. Clonage de l'ADN double brin

Pour supprimer les molécules d'ADN simple brin et obtenir un ADN double brin à extrémités franches, on traite les séquences non appariées par la nucléase S$_1$ selon la technique décrite par A. Efstradiatis et coll., Cell (1976), 7, p. 279 et suivantes. On sépare les ADNs néoformés double brin selon leur taille par centrifugation dans un gradient de saccharose. On utilise généralement un gradient de 5 % - 20 % de saccharose en 50 mM Tris-HCl pH 8,5, 10 mM EDTA , 800 mM NaCl, centrifugé à 210000 g pendant 15 heures, à 20°C, et on effectue un fractionnement du gradient en aliquots après centrifugation.

On contrôle la taille des molécules dans chaque fraction par électrophorèse d'échantillons faite en parallèle avec des étalons d'ADN de tailles connues, et l'on regroupe les fractions contenant un ADN constitué par l'enchaînement de plus de 500 paires de bases.

Pour permettre le clonage de cet ADN on allonge d'abord ses extrémités 3' avec de l'oligo(dC), et on allonge parallèlement les extrémités 3' du site PstI du plasmide vecteur pBR322 avec de l'oligo(dG) selon la technique de F. Rougeon et coll., J. Biol. Chem. (1977), 252, p. 2209 et suivantes.

On hybride alors l'ADN double brin décrit ci-dessus au plasmide vecteur, selon par exemple la technique de L. Villa-Komaroff et coll. Proc. Natl. Acad. Sci. (USA) (1978), 75, p. 3727 et suivantes.

On crée une "banque" de clones de cDNAs de foie par transformation de la bactérie E.coli avec l'ADN ainsi décrit selon la méthode décrite par M. Mandel et A. Higa, J. Mol. Biol. (1970), 53, p. 154 et suivantes et M. Dagert et S.D. Erlich., Gene (1979), 6, p. 23 et suivantes.

#### d. Repérage des clones de cDNA albumine

On utilise une technique d'hybridation sur colonies à l'aide d'oligonucléotides synthétiques dont les séquences sont déduites de la séquence protéique de l'albumine humaine (B. Meloun et al., FEBS Letters (1975), 58, p. 134 et suivantes ; M. Grunstein et D. Hogness, Proc. Natl. Acad. Sci. (USA) (1975), 72, p. 3961 et suivantes ; R.B. Wallace et coll., Nucleic Acids Res. (1981), 9, p. 879 et suivantes).

Les clones sont cultivés par séries de 96 sur milieu de Luria contenant 25 µg/ml de tétracycline, en boîtes carrées, directement sur des filtres de nitrocellulose. Après croissance à 37°C puis amplification en présence de 250 µg/ml de chloramphénicol, les colonies sont lysées par la soude puis hybridées avec les oligonucléotides radioactives en 5' par kination, dans une solution contenant : 5 X SSC, 0,5 % NP 40, 100 µg/ml ADN de sperme de saumon dénaturé par ébullition et refroidi rapidement dans la glace, 0,5 ng/ml d'oligonucléotide kinasé. L'hybridation est effectuée à 37°C pendant 18 heures. On lave ensuite les filtres en 5 X SSC, à 25°C, puis 37°C, puis 45°C et ce pendant quatre fois 15 minutes à chaque étape.

Les filtres sont alors exposés sur films Kodak X-OMAT, à -70°C, avec un écran amplificateur pendant 15 à 24 heures. Les clones hybridants avec les sondes sont réisolés puis lysés. L'ADN plasmidique est purifié par centrifugation en milieu chlorure de césium-bromure d'éthidium selon une technique connue.

On séquence l'ADN de l'insertion par la technique de Maxam-Gilbert (A. Maxam et W. Gilbert, Methods Enzymol. (1980), 65, p. 499 et suivantes) pour comparer la séquence protéique dérivée de la séquence nucléotidique et celle de la sérum-albumine humaine.

On identifie ainsi une série de clones dont les insertions correspondent à l'ensemble du gène de la sérum-albumine humaine.

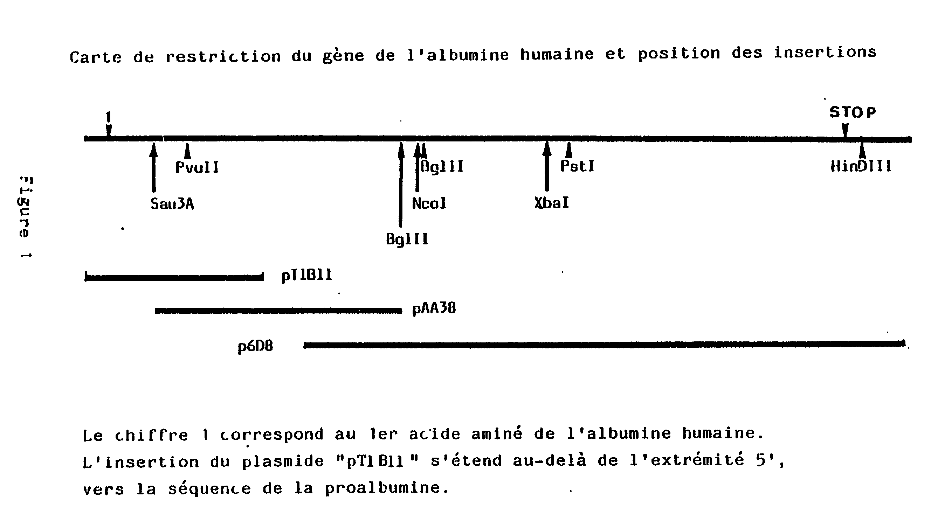

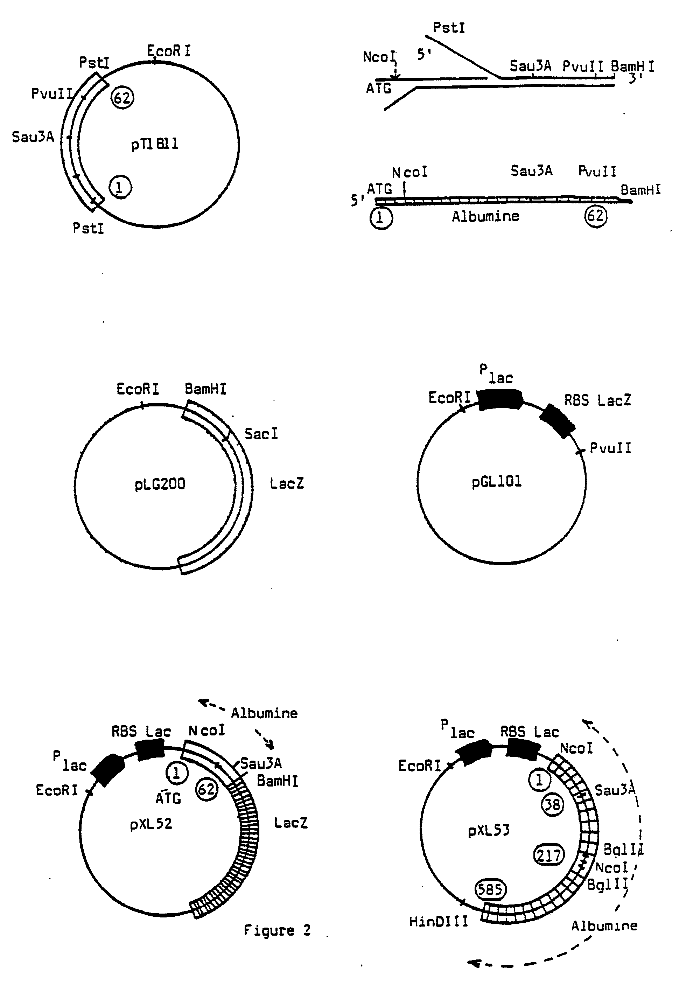

Dans la figure 1 est représentée la carte de restriction du gène de la sérum-albumine, ainsi que la position de trois des insertions les plus représentatives, désignées par "pT1B11", "pAA38". "p6D8".

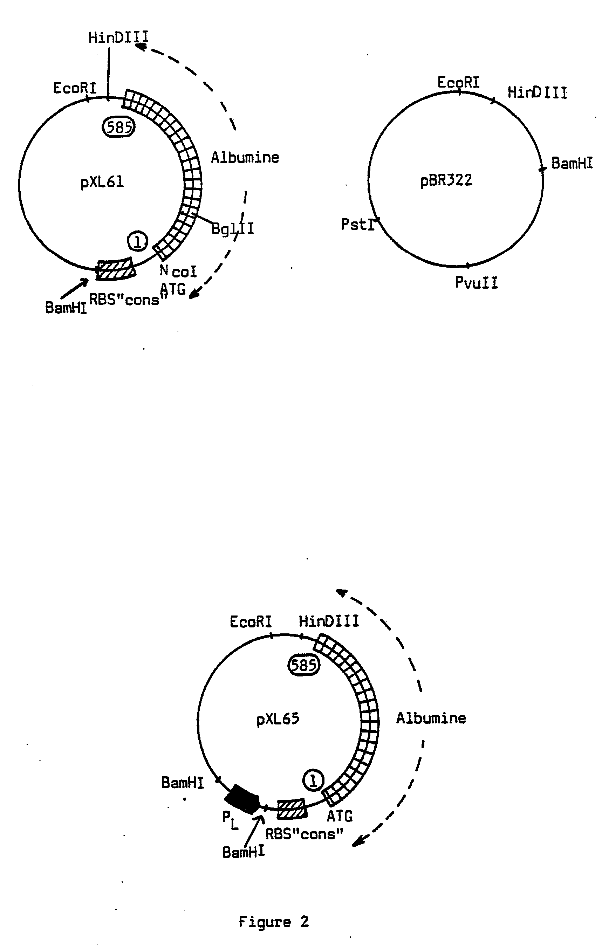

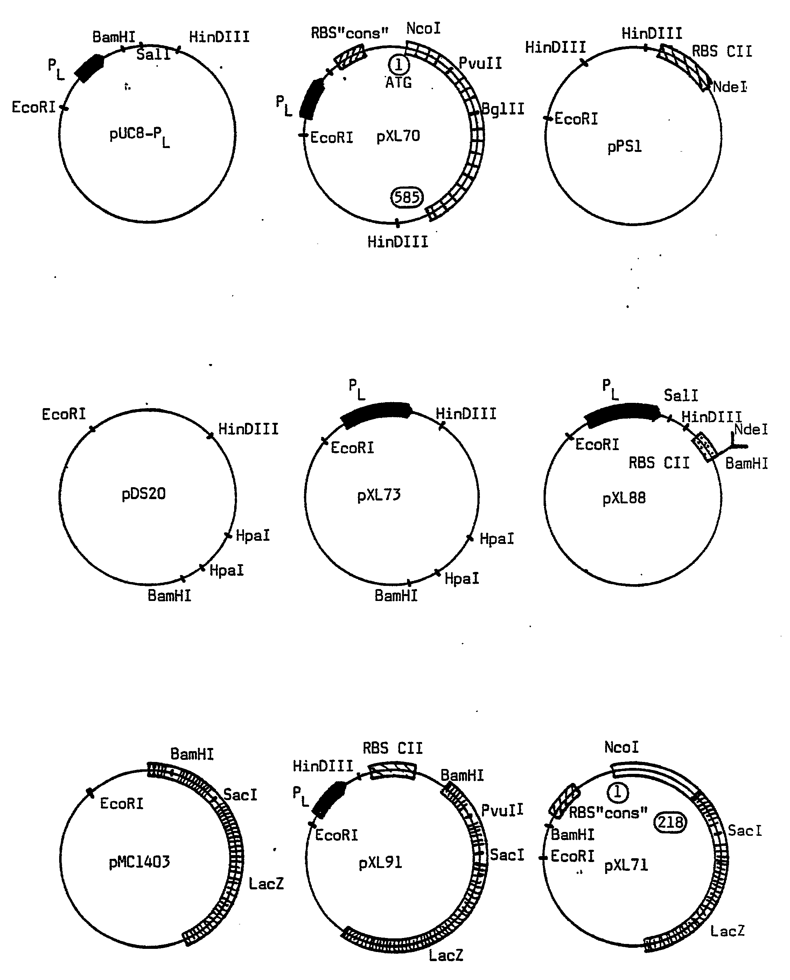

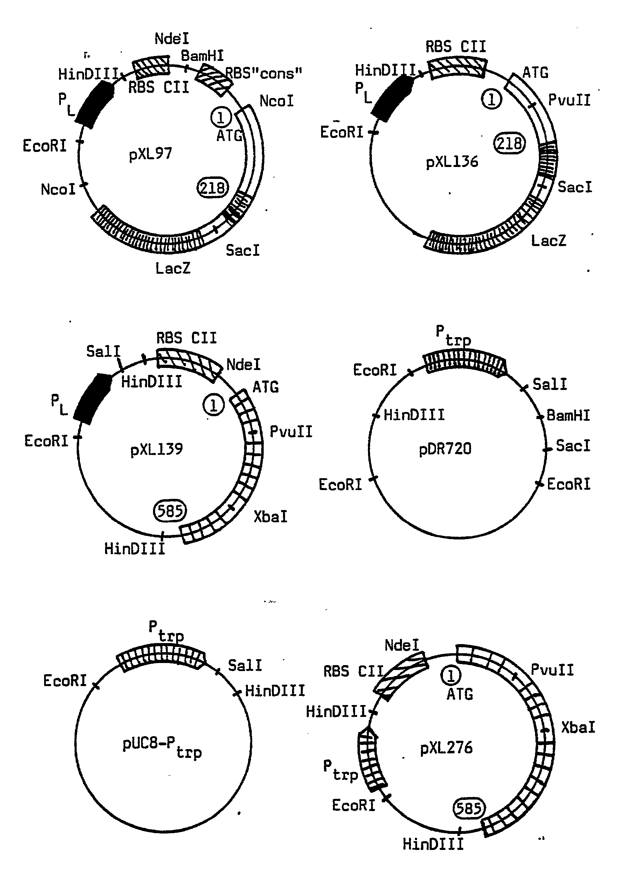

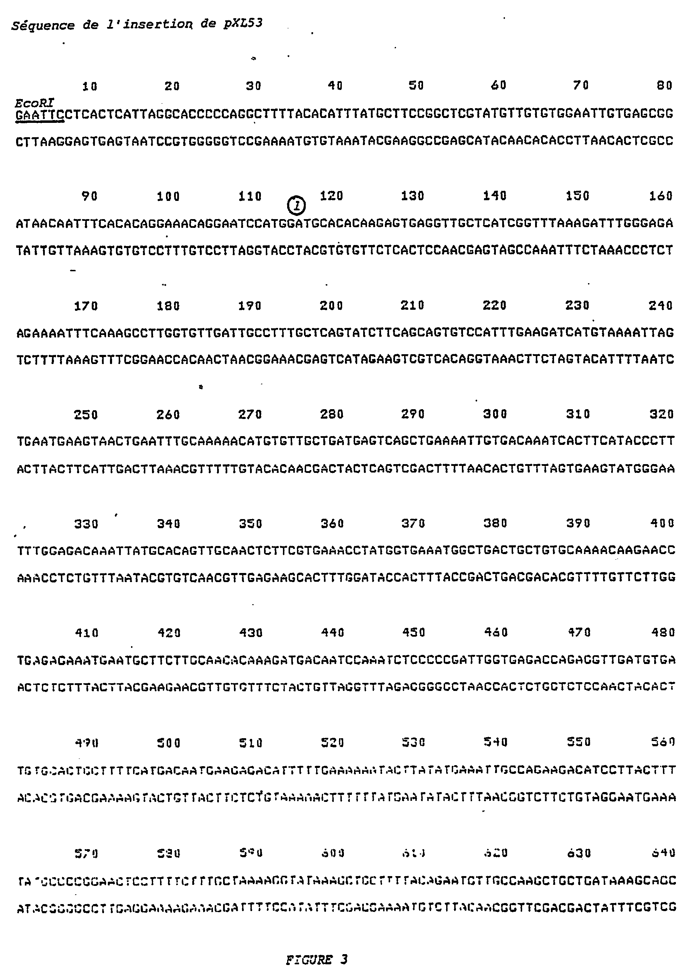

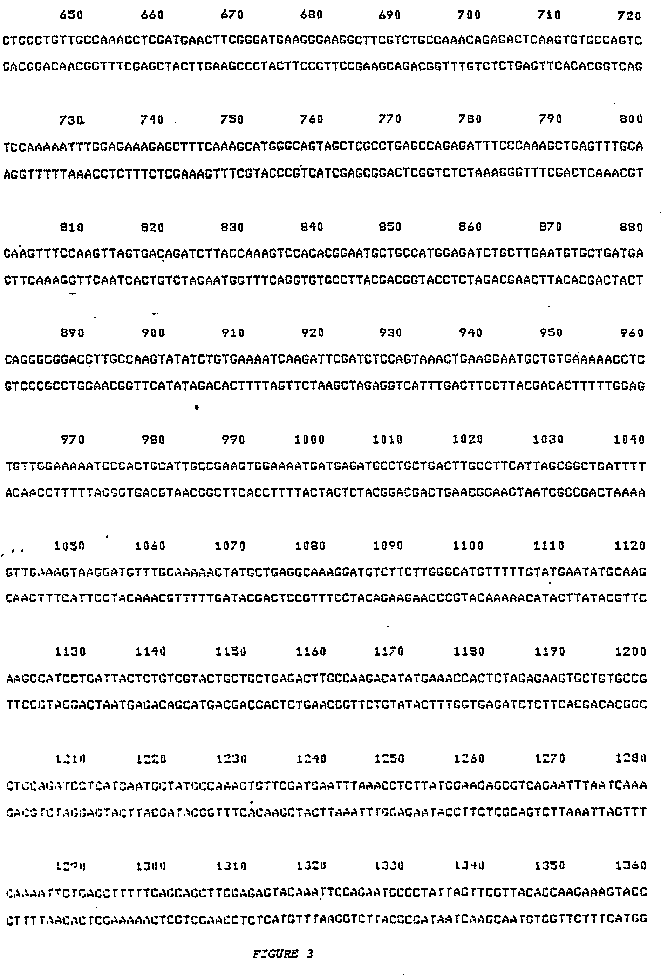

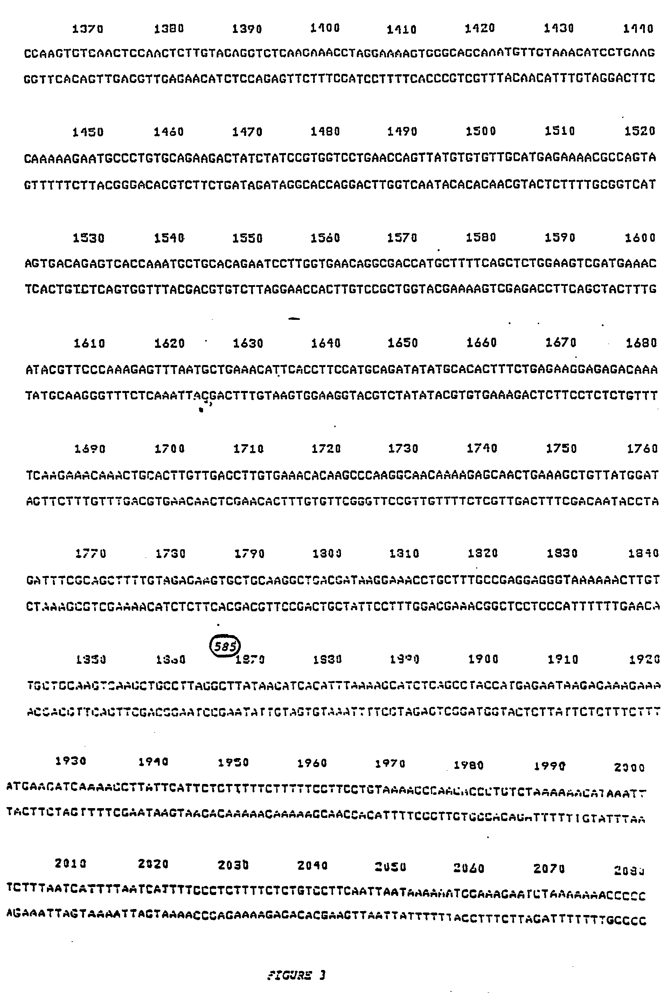

e. Incorporation au gène de structure d'un codon d'initiation (figure 2)

a) On digère l'ADN du plasmide "pT1B11" par les enzymes PstI et PvuII, et on isole un fragment d'ADN de 125 paires de bases, correspondant à la séquence de l'extrémité 5' du gène de la sérum-albumine (acides aminés n° 1 à 62). On fixe à l'extrémité PvuII une séquence de jonction constituée du site de reconnaissance de l'enzyme BamHI. On obtient ainsi un fragment PstI-BamHI.

On prépare d'autre part un oligonucléotide synthétique ayant 21 bases de long, possédant un triplet "ATG" devant les nucléotides codant pour les acides aminés de la sérum-albumine humaine ainsi qu'un site de restriction NcoI, et dont la séquence est la suivante : 5'GAATCCATGGATGCACACAAG 3'.

On dénature le fragment d'ADN PstI-BamHI, et on l'hybride avec l'oligonucléotide synthétique. L'hybridation se fait par la séquence 5'...GATGCACACAAG 3', l'extrémité 3' du brin d'ADN complémentaire étant désappariée. On digère les extrémités désappariées, puis on polymérise dans le sens 5'...3' avec le fragment "Klenow" de l'ADN polymérase I, d'après les techniques de H. Jacobsen et coll., Eur. J. Biochem. (1974), 45, p. 623 et suivantes.

On obtient ainsi un fragment contenant en 5' une extrémité franche, un site NcoI puis le triplet ATG et en 3' un site BamHI.

b) On réalise la ligation de trois fragments d'ADN :

1) un fragment EcoRI-BamHI du plasmide "pLG200" (L. Guarente et coll., Cell (1980) 20, p. 543 et suivantes) portant un gène de résistance aux antibiotiques, l'origine de réplication et l'extrémité 3' du gène de la β-galactosidase,

2) un fragment EcoRI-PvuII du plasmide "pGL101" (G. Lauer et coll., J. Mol. Appl. Genet. (1981), 1, p. 139 et suivantes) portant le promoteur $P_{lac}$ et le site de fixation de ribosome (RBS) du gène lacZ d'E.coli,

3) le fragment d'ADN mutagénisé codant pour les 62 premiers acides aminés de l'albumine humaine.

On isole un plasmide (pXL52) qui réalise une fusion de l'extrémité 5' du gène de la sérum-albumine humaine avec le gène de la β-galactosidase d'E.coli.

f. Construction du gène complet (figure 2)

On digère l'ADN du plasmide "p6D8" par EcoRI, et partiellement par BglII, selon une technique déjà décrite. On isole le grand fragment EcoRI-BglII contenant la séquence codant pour les 405 derniers acides aminés de la sérum-albumine humaine puis l'origine de replication du plasmide et le gène de résistance à la tétracycline.

On digère l'ADN du plasmide "pXL52" décrit ci-dessus par EcoRI et Sau3A, et on isole un fragment contenant 200 paires de bases.

On digère l'ADN du plasmide "pAA38" par Sau3A et on isole un fragment contenant 540 paires de bases.

On ligature les trois fragments (dans l'ordre [pXL52 EcoRI-Sau3A] - [pAA38 Sau3A] - [p6D8 BglII-EcoRI]) en tirant profit de la compatibilité entre les sites Sau3A et BglII. On obtient un plasmide appelé 'pXL53", dont la qualité de la construction est contrôlée par un séquençage complet du fragment compris entre le site EcoRI et le site PstI correspondant à la jonction de l'insertion et du plasmide vecteur.

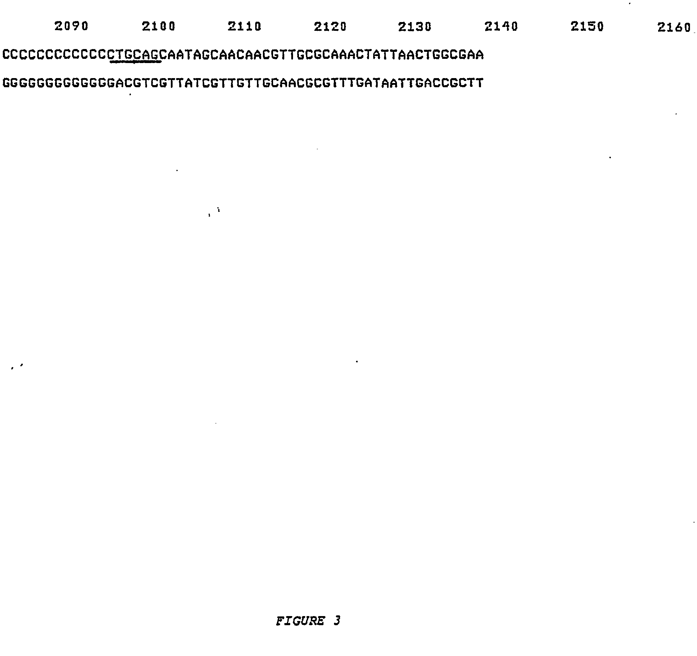

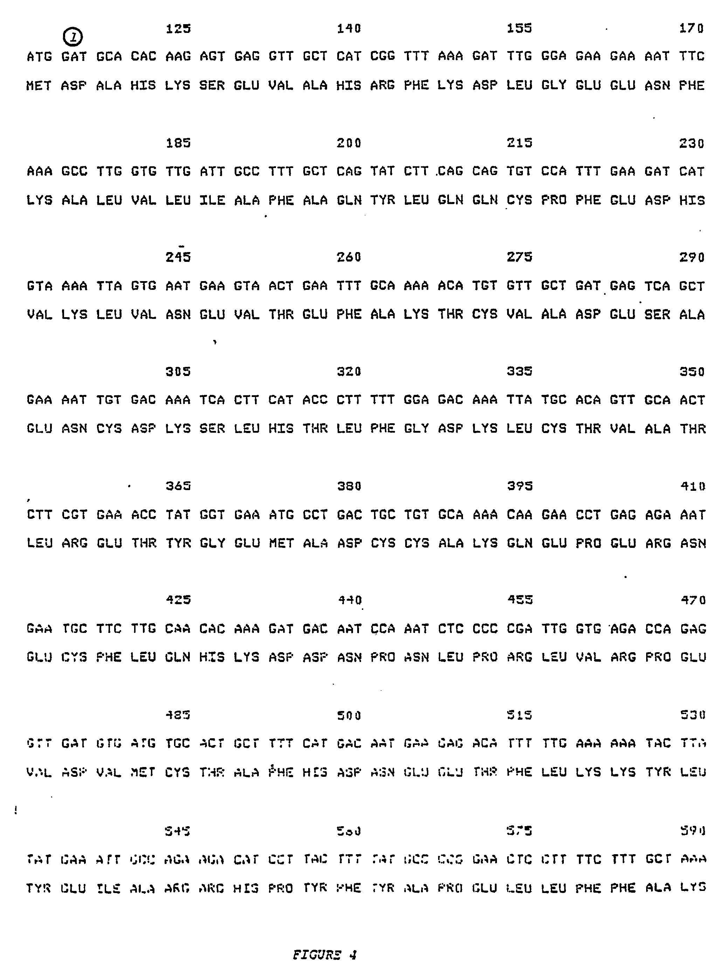

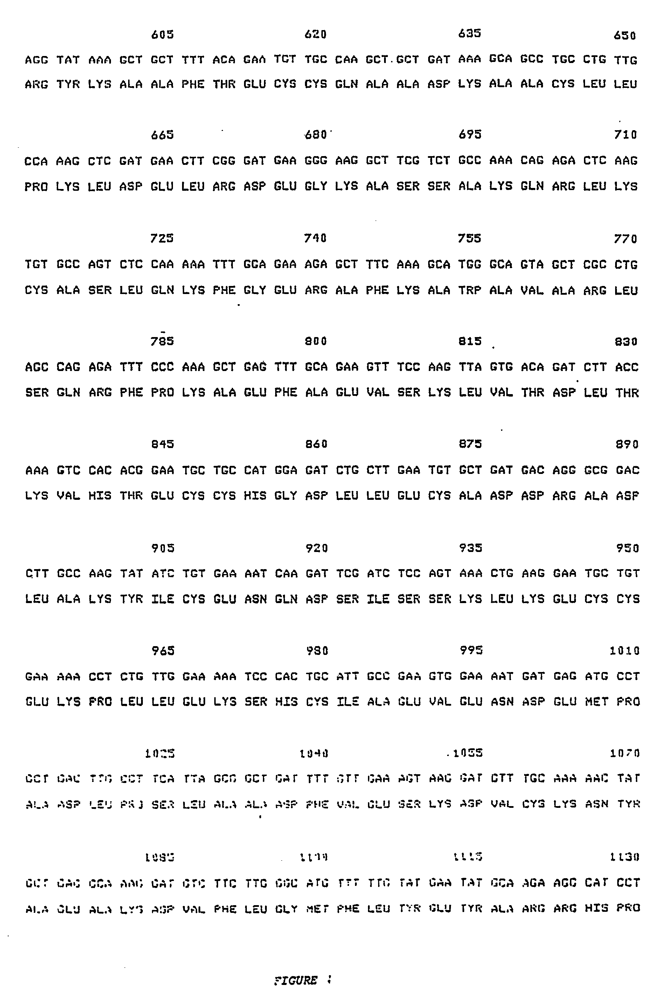

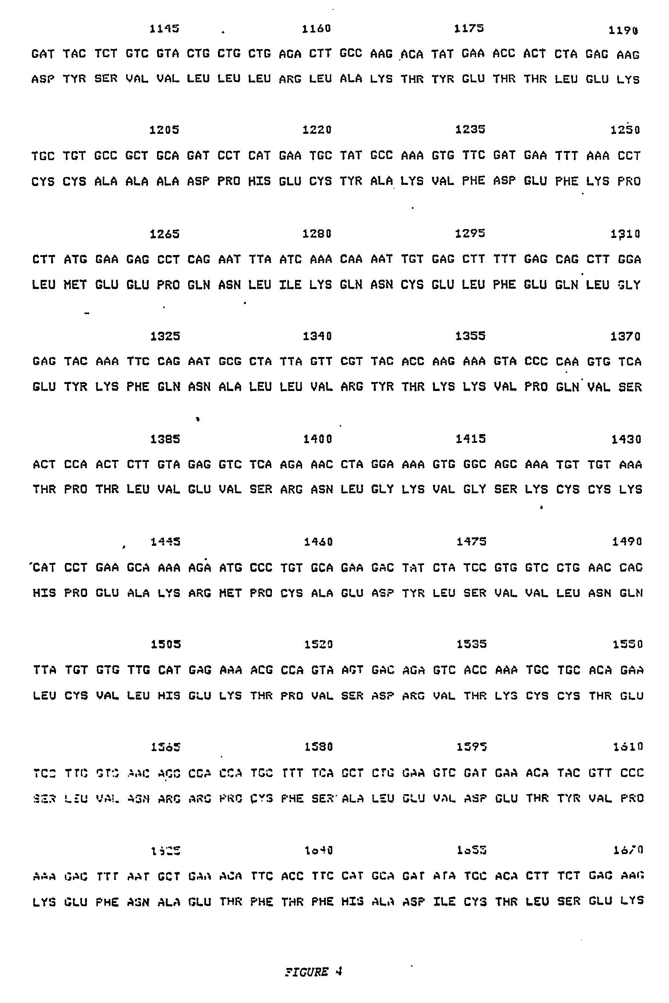

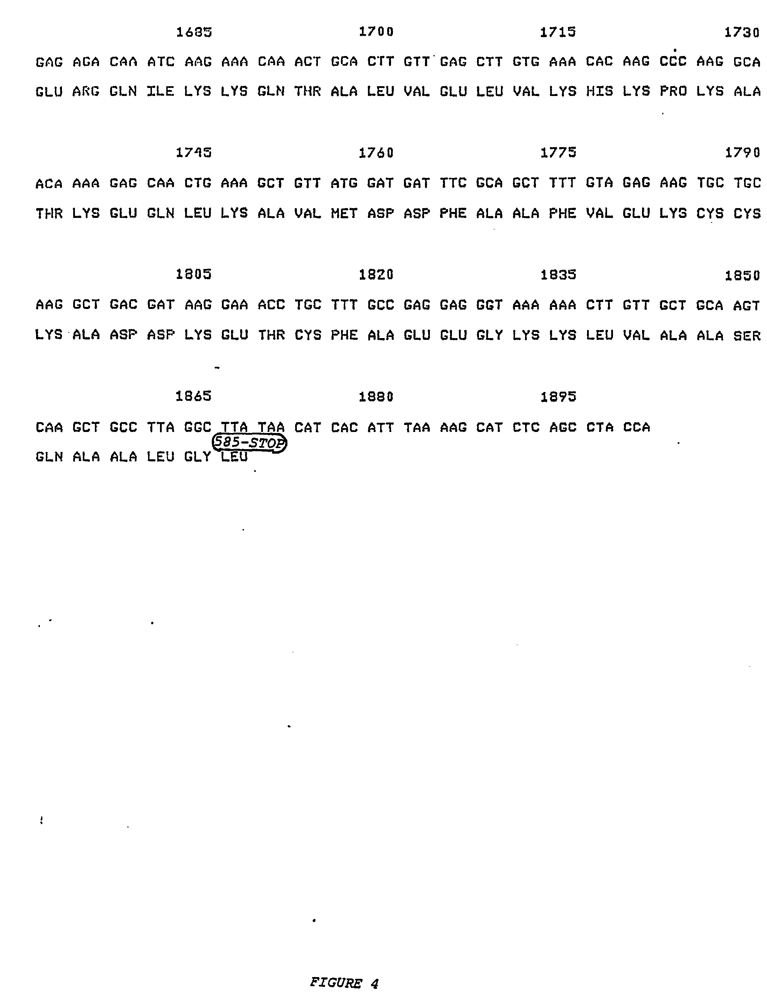

La séquence nucléotidique complète, ainsi que la séquence protéique dérivée, sont représentées dans les figures 3 et 4.

Les variations observées entre cette séquence et la séquence protéique publiée (B. Meloun et coll, FEBS Letters (1975), 58, p. 134 et suivantes ; M. Dayhoff, Atlas of Protein sequence and structure (1978), 5, supplément 3, p. 306) sont les suivantes :

| Position | Meloun et coll. | Sérum albumine humaine déduite de la séquence de "pXL53" |
|----------|-----------------|-----------------------------------------------------------|
| 131 | Glutamine | Acide glutamique |
| 364 | Histidine | Alanine |
| 367 | Tyrosine | Histidine |
| 370 | Alanine | Tyrosine |
| 381 | Valine | Méthionine |
| 464 | Acide glutamique | Histidine |
| 465 | Histidine | Acide glutamique |
| 501 | Glutamine | Acide glutamique |

B. CONSTRUCTION DE SYSTEMES D'EXPRESSION DE LA SERUM-ALBUMINE HUMAINE

1° Utilisation du promoteur "P$_L$" du bactériophage lambda

a) On linéarise le plasmide "pXL53" par digestion partielle par l'enzyme Ncol, en ne considérant que le site Ncol en 5' du codon d'initiation et on forme des bords francs par remplissage selon la technique de R.M. Wartell et W.S. Reznikoff, Gene (1980), 9, p. 307 et suivantes).

On synthétise un "adaptateur" contenant en 5' une séquence correspondant au site de reconnaissance d'une enzyme de restriction telle que BamHI, puis une séquence correspondant à un site de fixation de ribosomes (RBS "consensus" ou "théorique"). La séquence de l'adaptateur est : 5'GGATCCTAGGAGGAAC 3'.

La ligation de l'adaptateur en 5' d'un ADN à bords francs a été décrite, par exemple, par C.P. Bahl et coll., Gene (1976), 1, p. 81 et suivantes.

La méthode consiste à effectuer la réaction sur 20 microlitres d'une solution contenant 50 mM Tris, HCl pH = 7,5, 10 mM MgCl$_2$, 15 mM DTT, 1mM ATP, 50 µg/ml d'adaptateur, 20 µg/ml d'ADN et 1 unité d'ADN-ligase (New England Biolabs Inc.). La réaction est poursuivie pendant 10 heures à 15°C. Cette ligation crée un site BamHI sans supprimer le site Ncol.

On digère le produit de ligation par BamHI et par HinDIII. Du fait de la présence d'un site HinDIII en 3' du gène de la sérum-albumine humaine, on obtient un fragment d'ADN contenant la totalité de la séquence codante.

On sous-clone le fragment HinDIII-BamHI ainsi obtenu par exemple dans le plasmide "pBR322" en transformant E.coli selon la méthode déjà décrite ci-dessus pour obtenir le plasmide "pXL61".

Le plasmide "pXL61" ne contient pas de promoteur.

Le promoteur "P$_L$" du bactériophage lambda est placé sur le chromosome du bactériophage entre un site BglII et un site BamHI (voir E. Szybalski et W. Szybalski, Gene (1979) 7, p. 217 et suivantes), et dont la séquence nucléotidique est connue (F. Sanger et coll., J. Mol. Biol. (1982), 162, p. 279 et suivantes). On peut cloner ce fragment et modifier ses sites de restriction selon des méthodes connues.

On note que les plasmides portant P$_L$ doivent être propagés dans des souches de E.coli portant le gène répresseur cl, ceci afin d'éviter que ce promoteur ne s'exprime de façon constitutive.

Dans une première construction, P$_L$ est disponible sous forme d'un fragment BamHI à partir du plasmide "pPL-lambda" (Pharmacia P.L. Biochemicals). L'insertion de ce fragment BamHI dans le site BamHI du plasmide "pXL61" permet d'obtenir le plasmide "pXL65", dans lequel on a vérifié que l'orientation du promoteur par rapport au gène de structure de la sérum-albumine humaine est correcte.

D'autres constructions peuvent être réalisées à partir de plasmides disponibles. On peut, par exemple, exciser du plasmide "pPL-lambda" un fragment HaeIII-HaeIII contenant le promoteur P$_L$ et l'insérer dans le site SmaI d'une séquence de clonage multisite portée sur un plasmide, tel que le plasmide "pUC8" (J. Vieira et J. Messing, Gene, (1982), 79, p. 259 et suivantes) pour obtenir "pUC8-P$_L$"' dans lequel le site EcoRI est en 5' du promoteur.

A partir du plasmide "pPSI" (P. Sarmientos et coll., Cell (1983), 32, p. 1337 et suivantes), on peut d'abord détruire le site HinDIII le plus proche du site NdeI (figure 2) puis remplacer le petit fragment

EcoRI-HinDIII par, d'une part, le fragment EcoRI-BamHI du plasmide "pUC8-P$_L$" contenant le promoteur P$_L$, et, d'autre part, le fragment BamHI-HinDIII du plasmide "pXL61" contenant le gène de la sérum-albumine. On obtient ainsi le plasmide "pXL70" dans lequel l'ensemble P$_L$-RBS "consensus"-ATG-gène de la sérum-albumine humaine est porté sur un fragment d'ADN EcoRI-HinDIII.

b) Remplacement du RBS "consensus" par celui de gène CII du bactériophage lambda

Le gène CII du bactériophage lambda dont la séquence et le site d'initiation sont connus peut être traduit avec efficacité (E. Schwarz et coll., Nature (1978), 272, p. 410 et suivantes).

On construit un plasmide contenant le système d'expression "Promoteur "P$_L$" - RBS CII - ATG - gène sérum-albumine".

Par exemple, on peut après avoir détruit le site BamHI de "pUC8-P$_L$" par action de l'enzyme SI (A.J. Berk et P.A. Sharp, Cell (1977), 12, p. 721) isoler un fragment EcoRI-HinDIII contenant le promoteur P$_L$ et ensuite lier ce fragment avec un fragment EcoRI-HinDIII du plasmide "pDS20" (G. Duester et coll., Cell (1982), 30, p. 855 et suivantes, pour obtenir le plasmide "pXL73".

Le RBS du gène CII est extrait du plasmide "pPS1". On digère ce plasmide par NdeI et on insère un adaptateur BamHI après formation d'extrémités franches. On excise alors le RBS sous forme d'un fragment HinDIII-BamHI.

On construit d'abord un plasmide "pXL88" dans lequel ce fragment HinDIII-BamHI est lié au grand fragment HinDIII-BamHI du plasmide "pXL73". Dans le nouveau plasmide "pXL88", le RBS CII est inséré dans la bonne orientation rapport au promoteur P$_L$, le tout dans un système multisites de telle sorte que l'ensemble P$_L$-RBS CII soit porté sur un fragment d'ADN EcoRI-BamHI de 578 paires de bases.

Le fragment EcoRI-BamHI de 578 paires de bases est sous-cloné entre les sites EcoRI et BamHI du plasmide "pMC1403" (M.J. Casadaban et coll., J. Bacteriol. (1980), 143, p. 971 et suivantes) qui porte le gène de la β-galactosidase (lacZ) après le site BamHI. Cette construction conduit au plasmide "pXL91" dans lequel le gène de la β-galactosidase est exprimé sous contrôle du système "P$_L$-RBS CII".

On sous-clone le fragment BamHI-BglII du plasmide "pXL61" décrit précédemment dans le site BamHI du plasmide "pMC1403". (La ligation d'un site BglII dans un site BamHI est possible, mais l'excision par BamHI en BglII ne l'est plus ; il ne reste donc qu'un site BamHI).

Cette construction ("pXL71") aboutit à l'insertion d'un fragment d'ADN de 700 paires de bases comportant la séquence "BamHI-[RBS "consensus"-ATG-NcoI-gène partiel de la sérum-albumine (codant pour les acides aminés 1 à 218)-gène de la β-galactosidase].

On coupe ce plasmide par BamHI et SacI (le site SacI est présent dans le gène de la β-galactosidase) et on l'insère dans le plasmide "pXL91" décrit précédemment à la place du fragment préexistant BamHI-SacI.

On aboutit alors au plasmide "pXL97" dont l'insertion a la structure suivante :
"Site EcoRI - P$_L$ - RBS CII - site BamHI - RBS "consensus"- site NcoRI - ATG - gène partiel de la sérum-albumine - gène de la β-galactosidase".

On digère le plasmide "pXL97" par BamHI et partiellement par NcoI en ne considérant que le site NcoI proche du codon d'initiation et on forme les bords francs par action de la nucléase S1, puis on le referme sur lui-même. Cette manipulation, d'une part, supprime la séquence d'ADN du RBS "consensus" et, d'autre part, met en phase un ATG du RBS CII avec la séquence de la sérum-albumine.

On obtient ainsi le plasmide "pXL136" qui comporte la séquence "site EcoRI-P$_L$-RBS CII-ATG-gène partiel de la sérum-albumine-gène de la β-galactosidase".

Le gène partiel de la sérum-albumine possède un site PvuII, on digère le plasmide "pXL136" par Eco-RI et PvuII et on extrait un fragment de 760 paires de bases qui est inséré entre les sites EcoRI et PvuII du plasmide "pXL70" décrit précédemment. On obtient ainsi le plasmide "pXL139" qui porte la structure "P$_L$-RBS CII-gène sérum-albumine complet" sur un fragment EcoRI-HinDIII, comme le plasmide "pXL70" et qui porte la substitution RBS "consensus" par celui du gène CII.

c) Expression de la sérum-albumine après induction du promoteur "P$_L$"

On inocule une colonie isolée de E.coli portant le gène répresseur thermosensible du promoteur "P$_L$" (gène cI$^{ts}$) et transformée par l'un des plasmides "pXL65", "pXL70" et "pXL139".

Lorsque la bactérie est en phase exponentielle, on induit le promoteur "P$_L$" du plasmide en élevant très rapidement la température d'incubation à 42°C. On continue l'incubation pendant 90 minutes. On prélève un échantillon de la culture et on lyse la bactérie dans une suspension contenant 60 mM Tris-HCl pH = 6,8, 2 % SDS, 100 mM β-mercaptoéthanol, 10 % de glycérol et 0,1 % de bleu de bromophénol pendant 5 minutes.

On sépare les protéines par électrophorèse en gel de polyacrylamide selon la méthode de U.K. Laemli, Nature (1970), 227, p. 680 et suivantes ou de K. Weber et M. Osborne, J. Biol. Chem. (1969), 244, p. 4406 et suivantes.

On transfère les protéines sur un filtre de nitrocellulose (M. Bittner et coll., Anal. Biochem. (1980) 102, p. 459 et suivantes ; E.J. Stellwsag et A.E. Dahlberg, Nucleic Acid Res. (1980), 8, p. 229 et suivantes). On détecte la présence d'albumine humaine par immunologie, soit avec des anticorps anti-albumine humaine puis fixation de protéine A marquée, soit avec des anticorps anti-albumine biotinylés et révélés par des complexes avidineperoxydase.

On met ainsi en évidence la présence d'une protéine qui réagit avec des anticorps anti-albumine humaine, qui co-migre avec l'albumine authentique et qui n'est présente dans les lysats d'E.coli qu'après induction de cette bactérie à 42°C en présence du plasmide "pXL65", "pXL70" ou "pXL139".

On peut doser le taux de sérum-albumine humaine produite dans ces conditions. On obtient de façon reproductible une proportion d'albumine produite de l'ordre de 0,1 % de l'ensemble des protéines mises en évidence dans un lysat d'E.coli en conditions dénaturantes.

2° Utilisation du promoteur de l'opéron "tryptophane" de E.coli ($P_{trp}$) en remplacement du promoteur "$P_L$"

L'introduction du gène de structure de la sérum-albumine humaine derrière un promoteur bactérien inductible permet l'expression de cette protéine dans E.coli. Les taux d'expression des différents systèmes décrits précédemment sont voisins et de l'ordre de 1000 molécules de sérum-albumine par cellule. Ces résultats sont voisins de ceux obtenus avec des systèmes analogues tels qu'ils sont décrits dans les demandes de brevet européen EP 73646 et EP 91527. En particulier dans la demande de brevet européen EP 91527, il est indiqué une production maximale de 8000 molécules par cellule d'un "polypeptide semblable à la sérum-albumine humaine". La protéine obtenue n'est pas rigoureusement identique à la sérum-albumine humaine et les taux produits sont incompatibles avec les exigences de productivité industrielle. Par ailleurs, la production de sérum-albumine s'accompagne d'un effet létal sur la bactérie productrice.

Il a maintenant été trouvé, et c'est ce qui fait l'objet de la présente invention, que la production de la sérum-albumine humaine peut être considérablement améliorée en utilisant un plasmide contenant une association différente d'un promoteur inductible, du site de fixation de ribosomes d'un gène traduit avec efficacité et du gène de structure de la sérum-albumine humaine possédant en 5' un codon d'initiation ATG.

Plus particulièrement, l'invention concerne un procédé de préparation de la sérum-albumine humaine par culture d'une bactérie, telle qu'E.coli contenant un plasmide dans lequel le promoteur est celui de l'opéron "tryptophane" ($P_{trp}$) et le site de fixation de ribosomes celui du gène CII du bactériophage lambda ne contenant pas la séquence tRI en fin de transcription.

Le promoteur de l'opéron "tryptophane" d'E.coli permet d'induire l'expression d'un gène lorsque la souche d'E.coli est cultivée en absence de tryptophane ou en présence d'un analogue tel que l'acide indolyl-3 acrylique (C. Yanofsky et coll, Nucleic Acids Res. (1981), 9, p. 6647 et suivantes). Un tel promoteur est disponible sur des plasmides tels que "pDR720" (Société Pharmacia PL Biochemicals) (voir aussi D. Russel et G. Bennett, Gene (1982), 20, p. 231 et suivantes).

La construction d'un plasmide contenant le système "$P_{trp}$-RBS CII-gène sérum-albumine humaine" peut être réalisée de la manière suivante :

On insère le fragment EcoRI-SalI du plasmide "pDR270" contenant le promoteur $P_{trp}$ entre les sites EcoRI et SalI de "pUC8". On obtient alors le plasmide "pUC8-$P_{trp}$".

On coupe le plasmide "pXL139" décrit précédemment au site unique SalI, entre le promoteur $P_L$ et le RBS CII. On digère l'ADN par l'enzyme Bal31 de telle sorte que le site de fin de transcription tRI en 5' du RBS CII soit digéré puis on ajoute un adaptateur HinDIII et on isole le fragment HinDIII-XabI contenant le RBS CII amputé de tRI et les 357 premiers codons du gène de la sérum-albumine humaine. On combine ce fragment HinDIII-XbaI aveo, d'une part, le fragment XbaI-EcoRI du plasmide "pXL139" contenant la fin du gène de la sérum-albumine humaine et, d'autre part, le fragment EcoRI-HinDIII portant le promoteur $P_{trp}$ du plasmide "pUC8-$P_{trp}$".

On obtient ainsi le plasmide "pXL276". Ce plasmide ne contient plus que 41 nucléotides du RBS CII en amont du codon de démarrage ATG du gène de la sérum-albumine humaine.

## C. EXTRACTION ET RENATURATION DE LA SERUM-ALBUMINE HUMAINE SYNTHETISER PAR E.COLI

1° Culture de production

On utilise, par exemple, la souche G1381 qui dérive de la souche E.coli B 54125 (collection de l'Institut Pasteur) par transformation avec le plasmide "pXL276" suivant la méthode décrite par M. Mandel et A. Higa (J. Mol. Biol. (1970), 53, p. 154 et suivantes). Le plasmide "pXL276" contient le gène fonctionnel de la sérum-albumine humaine sous le contrôle du promoteur de l'opéron tryptophane ($P_{trp}$). L'expression de la sérum-albumine humaine est donc induite lorsque la bactérie est cultivée en absence de tryptophane ou en présence d'un analogue tel que l'acide 3-β-indolacrylique (B.P. Nichols et C. Yanofsky, Methods Enzymol. (1983), 101, p. 155 et suivantes).

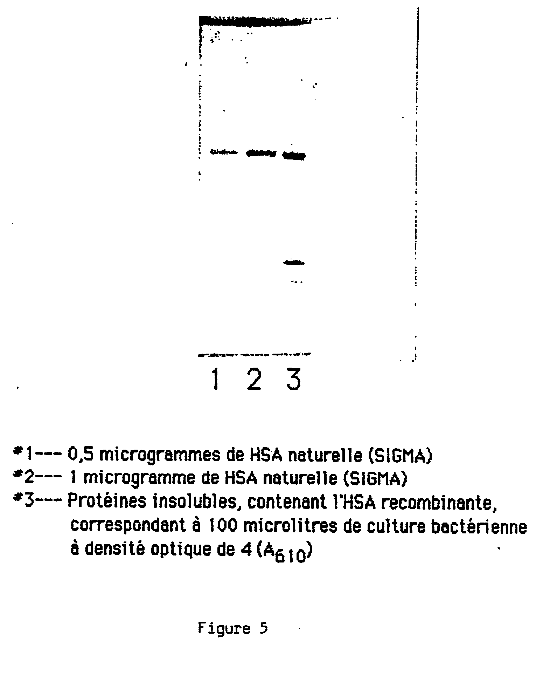

On peut procéder de la façon suivante. A partir d'un réisolement récent de la souche G1381 sur une boîte de Pétri gélosée à base de milieu LB (10 g de Bactotryptone (Difco), 5 g de Bacto Yeast Extract (Difco) et 5 g de NaCl par litre d'eau distillée) contenant 50 µg/ml d'ampicilline (LBAp), on prépare une préculture en milieu LBAp à 37°C avec agitation pendant 16 heures. Cette préculture est ensuite utilisée pour ensemencer au 1/100 un milieu synthétique sans tryptophane (par exemple milieu M9 glucosé + 0,1 % Casamino acids selon J.H. Miller, "Experiments in Molecular Genetics", Cold Spring Harbor Laboratory, New-York, (1972), p. 431 et suivantes). La culture de production est réalisée à 37°C de façon à optimiser le rendement de sérum-albumine humaine par litre de culture. Dans des conditions de laboratoire (culture en erlenmeyer), on récolte les cellules vers la fin de la phase exponentielle, après environ 6 heures de culture. La turbidité est alors comprise entre 3 et 4 unités de densité optique à 610 nm. Les rendements obtenus sont de l'ordre de 5 à 10 mg de sérum-albumine par litre de culture et par unité de densité optique à 610 nm, ce qui représente environ 5 à 7 % des protéines totales (voir figure 5).

2° Cassage des cellules et récupération de l'albumine insoluble

Après la culture de production, les cellules sont collectées et lysées, par exemple par centrifugation et sonication. A l'échelle du laboratoire, on peut utiliser un sonicateur Branson (Proscience, France, Modèle B30) après avoir concentré 20 à 30 fois les cellules dans le tampon PBS (0,2 g/l KCl, 0,2 g/l $KH_2PO_4$, 8 g/l NaCl et 1,25 g/l $Na_2HPO_4$). Le cassage des cellules est effectué à 0°C en mode continu (1 à 2 impulsions de 4 à 6 minutes suivant la densité cellulaire). On peut également prétraiter une suspension cellulaire concentrée en présence de 1 mg/ml de lysozyme de blanc d'oeuf pendant 10-20 minutes à température ambiante avant la sonication.

Dans ces conditions, la sérum-albumine humaine est presque entièrement insoluble ; il est donc possible d'obtenir à ce niveau une purification importante de cette protéine en centrifugeant le lysat cellulaire et en récupérant uniquement le culot de centrifugation obtenu après un cycle de lavage dans le tampon PBS. La sérum-albumine humaine représente alors de 30 à 50 % des protéines insolubles présentes dans le culot.

3° Solubilisation de la sérum-albumine humaine par dénaturation-réduction

La sérum-albumine ainsi obtenue peut être entièrement solubilisée, en conditions réductrices, par des concentrations élevées d'un agent chaotropique tel que l'urée 8M ou la guanidine-HCl 6M. D'après la littérature (G. Orsini et M.E. Goldberg, cf. supra), il est connu que de telles conditions assurent une dénaturation complète de la chaîne polypeptidique à l'état de pelote statistique et une réduction de tous les ponts disulfures inter- et intra-moléculaires. Plus particulièrement, si l'on reprend le "culot de sonication" décrit ci-dessus dans un tampon contenant 6M guanidine-HCl, 0,1M $KH_2PO_4$ pH 7,5, 0,1M β-mercaptoéthanol, de telle sorte que la sérum-albumine issue de <u>E.coli</u> soit présente à des concentrations de l'ordre de 1-5 mg/ml dans cette solution ("solution de dénaturation") et si on laisse la suspension obtenue sous agitation douce en récipient fermé pendant environ 16 heures à 4°C, on obtient une solution presque limpide. Les résidus insolubles peuvent alors être simplement éliminés par centrifugation ou filtration.

4° Renaturation de la sérum-albumine humaine obtenue par voie microbiologique

a. Détermination des conditions de renaturation

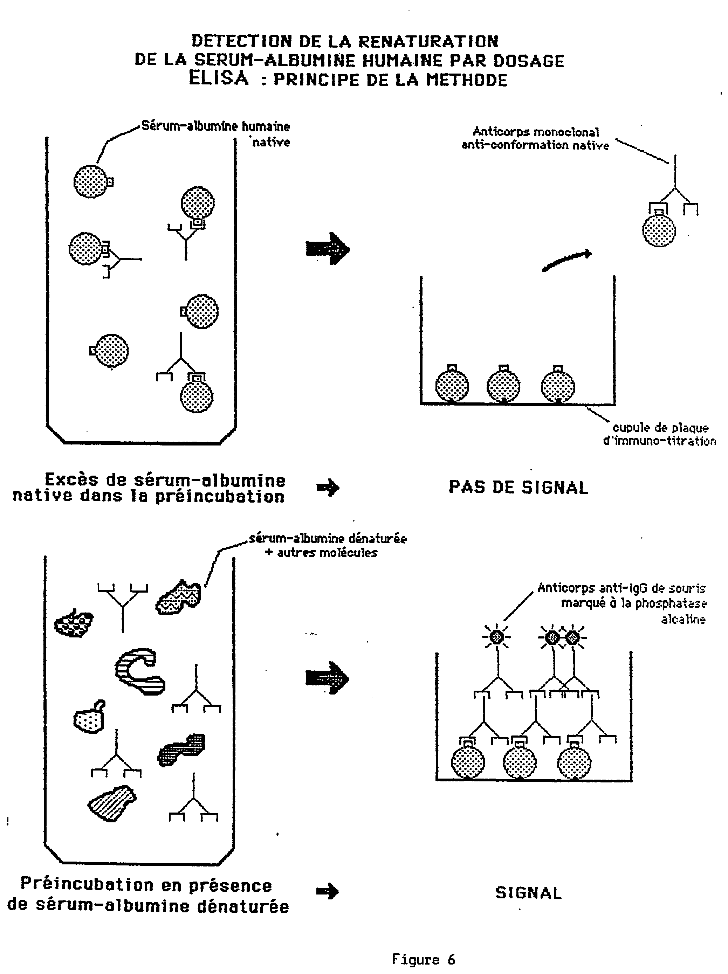

Pour permettre ensuite à la molécule de retrouver sa conformation native il est nécessaire d'éliminer ou de diminuer les concentrations des agents dénaturants et réducteurs. On peut, par exemple, effectuer une dilution de la fraction dénaturée/réduite dans des tampons permettant la renaturation. Il est possible d'évaluer le degré de renaturation de la protéine à l'aide d'une méthode de dosage ELISA selon la technique de L. Ohaniance et coll., (supra) en utilisant les anticorps monoclonaux dirigés contre la sérum-albumine décrits par Lapresle et Doyen (supra). Le principe d'une telle méthode est le suivant (figure 6). On fixe sur cupule la sérum-albumine native. Dans un autre tube, on mélange l'anticorps choisi pour l'analyse avec une gamme de concentrations du produit à examiner. Après incubation, le mélange est appliqué sur la cupule. La présence du motif antigénique correspondant à l'anticorps monoclonal dans le mélange initial inhibe la fixation de ce dernier à la sérum-albumine humaine accrochée sur la cupule. Dans une deuxième étape, on révèle la présence de l'anticorps monoclonal fixé sur la cupule à l'aide d'un deuxième anticorps couplé à une activité enzymatique : l'intensité du signal détecté sera donc, dans certaines conditions, inversement proportionnelle à la concentration en antigène spécifique dans la solution analysée. La description détaillée de la méthode est donné ci-après sous b.

Une préparation témoin de sérum-albumine commerciale dénaturée, réduite et traitée avec un excès de N-éthyl maléimide est employée pour distinguer un anticorps monoclonal spécifique de la séquence primaire de la sérum-albumine humaine d'un anticorps spécifique de la conformation native de la protéine. D'après le tableau 1, on peut constater, par exemple, que l'anticorps monoclonal HA4 est "spécifique de la séquence" tandis que HA9 reconnaît la protéine native sans reconnaître la sérum-albumine dénaturée et réduite. Egalement de ce tableau, il ressort que dans la suspension d'un culot de lysat soniqué d'une culture de la souche G1381 induite en milieu minimum, la présence de matériel antigénique est détectée par HA4 mais pas par HA9. Ceci est compatible avec l'hypothèse que la SAH fabriquée par E.coli n'est pas dans la conformation native caractéristique de la sérum-albumine d'origine naturelle.

TABLEAU 1 :

IDENTIFICATION DES ANTICORPS MONOCLONAUX SPECIFIQUES

DE LA CONFORMATION NATIVE DE LA SERUM-ALBUMINE :

EXEMPLE DE HA9

Pour une concentration donnée en antigène : $D.O._{420}$

|  | HA4 | HA9 |
|---|---|---|
| SAB native | 0,415 | 0,667 |
| SAH dénaturée, réduite traitée au NEM | 0,104 | 0,734 |
| SAH native | 0,063 | 0,007 |
| SAH coli avant renaturation | 0,032 | 0,810 |
| SAH coli après renaturation | 0,055 | 0,021 |

Abréviations : - SAB : sérum-albumine bovine

- SAH : sérum-albumine humaine

- NEM : N-éthylmaléimide

Si l'on dispose d'une méthode de dosage des antigènes caractéristiques de la conformation native, il devient possible d'estimer l'efficacité des conditions de renaturation exprimée sous la forme d'un rendement de renaturation. Ce rendement de renaturation est égal à R/T où R est la concentration en protéine renaturée calculée à partir d'une courbe de calibration obtenue avec le même monoclonal et la sérum-albumine native, et où T est la concentration totale en sérum-albumine d'origine microbiologique. Cette dernière concentration est calculée en comparant la coloration au bleu de Coomassié sur gel de polyacrylamide-SDS de la bande correspondant à la sérum-albumine dans l'échantillon considéré par rapport à une gamme de sérum-albumine dans les mêmes conditions.

On peut alors étudier les paramètres importants de la renaturation de la sérum-albumine humaine recombinante : nature du tampon utilisé, pH, force ionique, présence d'agents chélatants, température, durée de la renaturation, concentration optimale d'albumine pendant la phase de renaturation, etc...

Plus particulièrement, la renaturation de la sérum-albumine d'origine microbiologique peut être obtenue dans les conditions suivantes, retenues après une série d'optimisations :
50 mM Tris-HCl pH 8,5
100 mM NaCl
1 mM EDTA

Si on dilue 100 fois la sérum-albumine dénaturée et réduite dans cette "solution de renaturation", celle-ci reste soluble et on obtient au bout de 10-15 heures à 4°C un rendement de renaturation supérieur à 90 % si la concentration de sérum-albumine pendant la renaturation est inférieure à 50 mg/l. La solution ainsi obtenue présente une légère opalescence blanchâtre qui peut être éliminée par centrifugation ou filtration. Le précipité ainsi obtenu contient, en particulier, deux protéines de E.coli qui représentent la contamination protéique majeure de la sérum-albumine dans la fraction insoluble du lysat cellulaire. On réalise donc, en même temps que la renaturation, une étape de purification importante dans la mesure où la sérum-albumine représente maintenant 80 à 90 % du matériel protéique.

La sérum-albumine, entièrement présente dans la fraction soluble du mélange de renaturation, peut être concentrée par plusieurs méthodes ; certaines de ces méthodes peuvent servir simultanément d'étape de purification. Afin de pouvoir disposer d'un produit à une concentration compatible avec des analyses ultérieures, on peut également concentrer la sérum-albumine renaturée par ultrafiltration en utilisant les dispositifs commercialement disponibles. On obtient ainsi une solution contenant la sérum-albumine renaturée à une concentration de l'ordre de 1 mg/ml.

b. Description de la méthode de dosage ELISA

Les anticorps monoclonaux ont été fournis par le Professeur Lapresle (C. Lapresle et N. Doyen, supra).

On prépare comme suit une sérum-albumine humaine dénaturée pour servir de témoin négatif dans les expériences de renaturation. Après incubation pendant 16 heures à 4°C sous agitation douce, une solution de sérum-albumine humaine (Sigma) à 1 mg/ml dans la "solution de dénaturation" est diluée 100 fois dans la "solution de renaturation". On ajoute aussitôt 1/100 du volume de N-éthylmaléimide 0,5M (eau). La solution ainsi obtenue est dialysée contre le même tampon ("solution de renaturation") puis conservée à 4°C.

La sérum-albumine humaine native est adsorbée dans les cupules d'une plaque de micro-titration (Plaque Nunc Immuno type II) en incubant pendant 16 heures à 4°C les plaques contenant 200 $\mu$l par cupule d'une solution 0,1 mg/ml de la protéine dans un tampon carbonate de sodium 0,1M pH 9,5. Les plaques sont ensuites lavées trois fois par un tampon Tris-HCl 20 mM pH 7,5, 150 mM NaCl, 1 mM EDTA.

On détermine dans chaque cas la concentration optimale en anticorps monoclonal. Dans le cas des expériences décrites ci-dessus, les anticorps utilisés ont été dilués de 1/1000 à 1/2500 dans le tampon décrit ci-dessus.

On mélange 85 $\mu$l de la dilution d'anticorps avec un même volume de la solution de sérum-albumine à examiner. Le mélange est incubé à température ambiante pendant 15 minutes.

On dépose 100 $\mu$l de ce mélange dans une cupule prétraitée. La plaque est incubée pendant une heure à température ambiante.

Après lavage, on dépose 100 $\mu$l d'une dilution appropriée d'un deuxième anticorps (anti-1gG de souris couplé à la phosphatase alcaline ; Biosys, Compiègne, France). La plaque est de nouveau incubée pendant une heure à température ambiante.

Après lavage, on ajoute 150 $\mu$l de para-nitrophénylphosphate (PNPP) à 5,2 $\mu$g/ml dans un tampon 100 mM Tris-HCl pH 7,5 ; 1,35 M NaCl. La réaction enzymatique est stoppée par addition de 50 $\mu$l $K_2HPO_4$1M lorsque l'intensité de la couleur développée est suffisante. La densité optique est alors mesurée à 450 nm.

c. Analyse de la sérum-albumine humaine recombinante

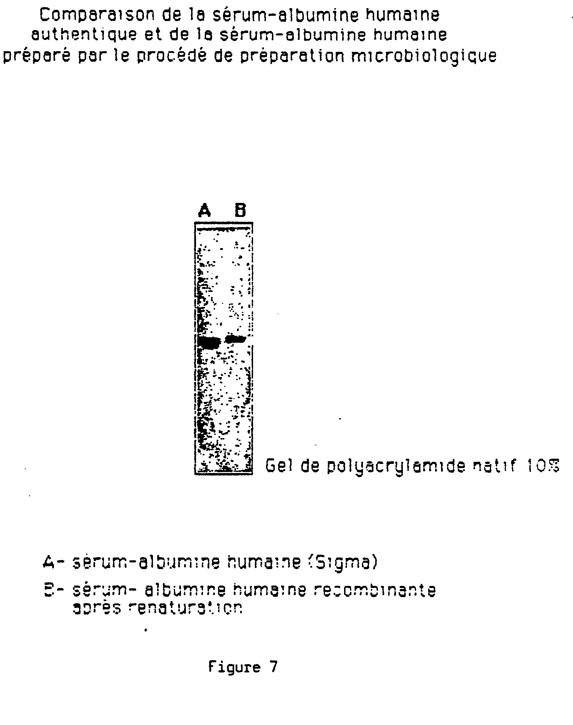

La sérum-albumine humaine recombinante obtenue après renaturation peut être analysée par diverses techniques décrites dans la littérature, comme l'électrophorèse sur gel de polyacrylamide, l'électrofocalisation ou différents types de chromatographies dans des conditions non dénaturantes qui rendent ces techniques très sensibles à la conformation de la protéine étudiée. On peut ainsi observer que cette protéine possède des caractéristiques très semblables au produit naturel (voir figure 7). D'autre part, le séquençage de l'extrémité N-terminale de la protéine ainsi produite indique la présence majoritaire d'une méthionine comme premier résidu.

La sérum-albumine humaine produite par <u>E.coli</u> et préparée par le procédé selon l'invention ne diffère de la sérum-albumine humaine authentique que par la présence d'une méthionine à l'extrémité N-terminale. Cette protéine est de plus obtenue avec un rendement nettement amélioré et sans effet létal sur la bactérie productrice.

Un échantillon du microorganisme E. Coli B, G1151 contenant le plasmide pXL 276 a été déposé au CBS à Baarn (Pays-Bas) sous le numéro CBS 151.86 conformément aux dispositions du traité de Budapest.

**Revendications**

1. Procédé de préparation de la sérum albumine humaine à l'état de chaîne peptidique dénaturée et insoluble, qui est transformée en protéine renaturée et soluble par applications de méthodes connues, caractérisé en ce que l'on cultive une bactérie choisie parmi les souches d'E.coli capables d'assurer le maintien d'un plasmide contenant le promoteur de l'opéron trytophane $P_{trp}$, le site de fixation des ribosomes du gène cII du bactériophage lambda ne contenant pas la séquence tRI de fin de transcription et le gène de la sérum albumine humaine contenant en 5' un codon d'initiation ATG.

2. Le plasmide pXL276 caractérisé en ce qu'il contient le promoteur $P_{trp}$, le site de fixation des ribosomes du gène cII du bactériophage lambda ne contenant pas la séquence tRI de fin de transcription et le gène de la sérum albumine humaine possédant en 5' un codon d'initiation ATG (numéro de dépôt de la souche d'E.coli contenant le plasmide pXL276: CBS-151–86).

## Claims

1. Process for preparing human serum albumin in the state of a denatured, insoluble peptide chain, which is converted to a renatured, soluble protein by applications of known methods, characterized in that a bacterium chosen from E.coli strains capable of providing for the maintenance of a plasmid containing the promoter of the tryptophan operon $P_{trp}$, the ribosome binding site of the cII gene of bacteriophage lambda not containing the tRI transcription termination sequence and the human serum albumin gene containing an ATG initiation codon at the 5′ end is cultured.

2. Plasmid pXL276, characterized in that it contains the $P_{trp}$ promoter, the ribosome binding site of the cII gene of bacteriophage lambda not containing the tRI transcription termination sequence and the human serum albumin gene possessing an ATG initiation codon at the 5′ end (deposition number of the E.coli strain containing plasmid pXL276: CBS-151–86).

## Patentansprüche

1. Verfahren zur Herstellung von menschlichem Serumalbumin im Zustand der denaturierten und unlöslichen Peptidkette, die durch Anwendung bekannter Methoden in renaturiertes und lösliches Protein umgewandelt wird, dadurch gekennzeichnet, daß man ein Bakterium züchtet, ausgewählt aus den Stämmen von E.coli, die den Bestand eines den Promotor des Tryptophan-Operons $P_{trp}$ enthaltenden Plasmids zu gewährleisten vermögen, wobei die Bindestelle der Ribosomen des Gens cII des Bakteriophagen Lambda die Transkriptions-Terminationssequenz tRI nicht enthält und das Gen des menschlichen Serumalbumins in 5′ ein ATG-Startcodon enthält.

2. Plasmid pXL276, dadurch gekennzeichnet, daß es den Prom $P_{trp}$ enthält, wobei die Bindestelle der Ribosomen des Gens cII des Bakteriophagen Lambda die Transkriptions-Terminationssequenz tRI nicht enthält und das Gen des menschlichen Serumalbumins in 5′ ein ATG-Startcodon enthält. (Hinterlegungs-Nummer des das Plasmid pXL276 enthaltenden Stammes von E.coli: CBS-151–86).

Carte de restriction du gène de l'albumine humaine et position des insertions

EP 0 198 745 B1

Figure 1

Le chiffre 1 correspond au 1er acide aminé de l'albumine humaine.
L'insertion du plasmide "pT1B11" s'étend au-delà de l'extrémité 5',
vers la séquence de la proalbumine.

Figure 2

Figure 2

EP 0 198 745 B1

*Séquence de l'insertion de pXL53*

```
          10        20        30        40        50        60        70        80
EcoRI
GAATTCCTCACTCATTAGGCACCCCCAGGCTTTTACACATTTATGCTTCCGGCTCGTATGTTGTGTGGAATTGTGAGCGG
CTTAAGGAGTGAGTAATCCGTGGGGGTCCGAAAATGTGTAAATACGAAGGCCGAGCATACAACACACCTTAACACTCGCC


          90       100       110    ①   120       130       140       150       160
ATAACAATTTCACACAGGAAACAGGAATCCATGGATGCACACAAGAGTGAGGTTGCTCATCGGTTTAAAGATTTGGGAGA
TATTGTTAAAGTGTGTCCTTTGTCCTTAGGTACCTACGTGTGTTCTCACTCCAACGAGTAGCCAAATTTCTAAACCCTCT


         170       180       190       200       210       220       230       240
AGAAAATTTCAAAGCCTTGGTGTTGATTGCCTTTGCTCAGTATCTTCAGCAGTGTCCATTTGAAGATCATGTAAAATTAG
TCTTTTAAAGTTTCGGAACCACAACTAACGGAAACGAGTCATAGAAGTCGTCACAGGTAAACTTCTAGTACATTTTAATC


         250       260       270       280       290       300       310       320
TGAATGAAGTAACTGAATTTGCAAAAACATGTGTTGCTGATGAGTCAGCTGAAAATTGTGACAAATCACTTCATACCCTT
ACTTACTTCATTGACTTAAACGTTTTTGTACACAACGACTACTCAGTCGACTTTTAACACTGTTTAGTGAAGTATGGGAA


         330       340       350       360       370       380       390       400
TTTGGAGACAAATTATGCACAGTTGCAACTCTTCGTGAAACCTATGGTGAAATGGCTGACTGCTGTGCAAAACAAGAACC
AAACCTCTGTTTAATACGTGTCAACGTTGAGAAGCACTTTGGATACCACTTTACCGACTGACGACACGTTTTGTTCTTGG


         410       420       430       440       450       460       470       480
TGAGACAAATGAATGCTTCTTGCAACACAAAGATGACAATCCAAATCTCCCCCGATTGGTGAGACCAGACGTTGATGTGA
ACTCTCTTTACTTACGAAGAACGTTGTGTTTCTACTGTTAGGTTTAGACGGGCCTAACCACTCTGGTCTCCAACTACACT


         490       500       510       520       530       540       550       560
TGTGCACTGCTTTTCATGACAATGAAGAGACATTTTTGAAAAATACTTATATGAAATTGCCAGAAGACATCCTTACTTT
ACACGTGACGAAAAGTACTGTTACTTCTCTGTAAAAACTTTTTTATGAATATACTTTAACGGTCTTCTGTAGGAATGAAA


         570       580       590       600       610       620       630       640
TATGCCCCCGGAACTCCTTTTCTTTGCTAAAACGTATAAAGCTGCTTTTACAGAATGTTGCCAAGCTGCTGATAAAGCACC
ATACGGGGGCCTTGAGGAAAAGAAACGATTTTGCCATATTTCGACGAAAATGTCTTACAACGGTTCGACGACTATTTCGTCG
```

*FIGURE 3*

EP 0 198 745 B1

```
        650       660       670       680       690       700       710       720
CTGCCTGTTGCCAAAGCTCGATGAACTTCGGGATGAAGGGAAGGCTTCGTCTGCCAAACAGAGACTCAAGTGTGCCAGTC
GACGGACAACGGTTTCGAGCTACTTGAAGCCCTACTTCCCTTCCGAAGCAGACGGTTTGTCTCTGAGTTCACACGGTCAG


        730       740       750       760       770       780       790       800
TCCAAAAATTTGGAGAAAGAGCTTTCAAAGCATGGGCAGTAGCTCGCCTGAGCCAGAGATTTCCCAAAGCTGAGTTTGCA
AGGTTTTTAAACCTCTTTCTCGAAAGTTTCGTACCCGTCATCGAGCGGACTCGGTCTCTAAAGGGTTTCGACTCAAACGT


        810       820       830       840       850       860       870       880
GAAGTTTCCAAGTTAGTGACAGATCTTACCAAAGTCCACACGGAATGCTGCCATGGAGATCTGCTTGAATGTGCTGATGA
CTTCAAAGGTTCAATCACTGTCTAGAATGGTTTCAGGTGTGCCTTACGACGGTACCTCTAGACGAACTTACACGACTACT


        890       900       910       920       930       940       950       960
CAGGGCGGACCTTGCCAAGTATATCTGTGAAAATCAAGATTCGATCTCCAGTAAACTGAAGGAATGCTGTGAAAAACCTC
GTCCCGCCTGCAACGGTTCATATAGACACTTTTAGTTCTAAGCTAGAGGTCATTTGACTTCCTTACGACACTTTTTGGAG


        970       980       990      1000      1010      1020      1030      1040
TGTTGGAAAAATCCCACTGCATTGCCGAAGTGGAAAATGATGAGATGCCTGCTGACTTGCCTTCATTAGCGGCTGATTTT
ACAACCTTTTTAGGGTGACGTAACGGCTTCACCTTTTACTACTCTACGGACGACTGAACGGAACTAATCGCCGACTAAAA


       1050      1060      1070      1080      1090      1100      1110      1120
GTTGAAGTAAGGATGTTTGCAAAAACTATGCTGAGGCAAAGGATGTCTTCTTGGGCATGTTTTTGTATGAATATGCAAG
CAACTTTCATTCCTACAAACGTTTTTGATACGACTCCGTTTCCTACAGAAGAACCCGTACAAAAACATACTTATACGTTC


       1130      1140      1150      1160      1170      1190      1190      1200
AAGCCATCCTGATTACTCTGTCGTACTGCTGCTGAGACTTGCCAAGACATATGAAACCACTCTAGAGAAGTGCTGTGCCG
TTCCGTAGGACTAATGAGACAGCATGACGACGACTCTGAACGGTTCTGTATACTTTGGTGAGATCTCTTCACGACACGGC


       1210      1220      1230      1240      1250      1260      1270      1280
CTCCAGATCCTCATGAATGCTATGCCAAAGTGTTCGATGAATTTAAACCTCTTATGGAAGAGCCTCAGAATTTAATCAAA
GAGGTCTAGGAGTACTTACGATACGGTTTCACAAGCTACTTAAATTTGGAGAATACCTTCTCGGAGTCTTAAATTAGTTT


       1290      1300      1310      1320      1330      1340      1350      1360
CAAAATTCTCAGCTTTTTCAGCCAGCTTGGAGAGTACAAATTCCAGAATGCCCTATTAGTTCGTTACACCAAGAAAGTACC
GTTTTAAGAGTCGAAAAAGTCGGTCGAACCTCTCATGTTTAAGGTCTTACGCCGATAATCAAGCAATGTGGTTCTTTCATGG
```

*FIGURE 3*

```
      1370      1380      1390      1400      1410      1420      1430      1440
CCAAGTCTCAACTCCAACTCTTGTACAGGTCTCAAGAAACCTAGGAAAAGTGGGCAGCAAATGTTGTAAACATCCTGAAG
GGTTCACAGTTGACGTTGAGAACATCTCCAGAGTTCTTTCCATCCTTTTCACCCGTCGTTTACAACATTTGTAGGACTTC


      1450      1460      1470      1480      1490      1500      1510      1520
CAAAAAGAATGCCCTGTGCAGAAGACTATCTATCCGTGGTCCTGAACCAGTTATGTGTGTTGCATGAGAAAACGCCAGTA
GTTTTTCTTACGGGACACGTCTTCTGATAGATAGGCACCAGGACTTGGTCAATACACACAACGTACTCTTTTGCGGTCAT


      1530      1540      1550      1560      1570      1580      1590      1600
AGTGACAGAGTCACCAAATGCTGCACAGAATCCTTGGTGAACAGGCGACCATGCTTTTCAGCTCTGGAAGTCGATGAAAC
TCACTGTCTCAGTGGTTTACGACGTGTCTTAGGAACCACTTGTCCGCTGGTACGAAAAGTCGAGACCTTCAGCTACTTTG


      1610      1620      1630      1640      1650      1660      1670      1680
ATACGTTCCCAAAGAGTTTAATGCTGAAACATTCACCTTCCATGCAGATATATGCACACTTTCTGAGAAGGAGAGACAAA
TATGCAAGGGTTTCTCAAATTACGACTTTGTAAGTGGAAGGTACGTCTATATACGTGTGAAAGACTCTTCCTCTCTGTTT


      1690      1700      1710      1720      1730      1740      1750      1760
TCAAGAAACAAACTGCACTTGTTGAGCTTGTGAAACACAAGCCCAAGGCAACAAAAGAGCAACTGAAAGCTGTTATGGAT
AGTTCTTTGTTTGACGTGAACAACTCGAACACTTTGTGTTCGGGTTCCGTTGTTTTCTCGTTGACTTTCGACAATACCTA


      1770      1780      1790      1800      1810      1820      1830      1840
GATTTCGCAGCTTTTGTAGAGAAGTGCTGCAAGGCTGACGATAAGGAAACCTGCTTTGCCGAGGAGGGTAAAAAACTTGT
CTAAAGCGTCGAAAACATCTCTTCACGACGTTCCGACTGCTATTCCTTTGGACGAAACGGCTCCTCCCATTTTTTGAACA


                              (585)
      1850      1860      1870      1880      1890      1900      1910      1920
TGCTGCAAGTCAAGCTGCCTTAGGCTTATAACATCACATTTAAAAGCATCTCAGCCTACCATGAGAATAAGAGAAAGAAA
ACGACGTTCAGTTCGACGGAATCCGAATATTGTAGTGTAAATTTTCCTAGAGTCGGATGGTACTCTTATTCTCTTTCTTT


      1930      1940      1950      1960      1970      1980      1990      2000
ATGAAGATCAAAACCTTATTCATTCTCTTTTTTCTTTTTCCTTCCTGTAAAAGCCAAGACCCTGTCTAAAAAACATAAATT
TACTTCTAGTTTTTCGAATAAGTAAGACAAAAACAAAAAGCAACCACATTTTCCCTTGTGGCACAGTTTTTTTGTATTTAA


      2010      2020      2030      2040      2050      2060      2070      2080
TCTTTAATCATTTTAATCATTTTCCCTCTTTTTCTCTGTGCTTCAATTAATAAAAAATGCAAAGAATCTAAAAAAACCCCC
AGAAATTAGTAAAATTAGTAAAACCCAGAAAAGAGACACGAAGTTAATTATTTTTTTACCTTTCTTAGATTTTTTTGGCCC
```

*FIGURE 3*

2090      2100      2110      2120      2130      2140      2150      2160

CCCCCCCCCCCCCCTGCAGCAATAGCAACAACGTTGCGCAAACTATTAACTGGCGAA

GGGGGGGGGGGGGGACGTCGTTATCGTTGTTGCAACGCGTTTGATAATTGACCGCTT

*FIGURE 3*

         ①         125                    140                    155              ·         170
ATG GAT GCA CAC AAG AGT GAG GTT GCT CAT CGG TTT AAA GAT TTG GGA GAA GAA AAT TTC
MET ASP ALA HIS LYS SER GLU VAL ALA HIS ARG PHE LYS ASP LEU GLY GLU GLU ASN PHE


                   185                    200                    215                        230
AAA GCC TTG GTG TTG ATT GCC TTT GCT CAG TAT CTT CAG CAG TGT CCA TTT GAA GAT CAT
LYS ALA LEU VAL LEU ILE ALA PHE ALA GLN TYR LEU GLN GLN CYS PRO PHE GLU ASP HIS


                   245                    260                    275                        290
GTA AAA TTA GTG AAT GAA GTA ACT GAA TTT GCA AAA ACA TGT GTT GCT GAT GAG TCA GCT
VAL LYS LEU VAL ASN GLU VAL THR GLU PHE ALA LYS THR CYS VAL ALA ASP GLU SER ALA


                   305                    320                    335                        350
GAA AAT TGT GAC AAA TCA CTT CAT ACC CTT TTT GGA GAC AAA TTA TGC ACA GTT GCA ACT
GLU ASN CYS ASP LYS SER LEU HIS THR LEU PHE GLY ASP LYS LEU CYS THR VAL ALA THR


             ·     365                    380                    395                        410
CTT CGT GAA ACC TAT GGT GAA ATG CCT GAC TGC TGT GCA AAA CAA GAA CCT GAG AGA AAT
LEU ARG GLU THR TYR GLY GLU MET ALA ASP CYS CYS ALA LYS GLN GLU PRO GLU ARG ASN


                   425                    440                    455                        470
GAA TGC TTC TTG CAA CAC AAA GAT GAC AAT CCA AAT CTC CCC CGA TTG GTG AGA CCA GAG
GLU CYS PHE LEU GLN HIS LYS ASP ASP ASN PRO ASN LEU PRO ARG LEU VAL ARG PRO GLU


                   485                    500                    515                        530
GTT GAT GTG ATG TGC ACT GCT TTT CAT GAC AAT GAA GAG ACA TTT TTG AAA AAA TAC TTG
VAL ASP VAL MET CYS THR ALA PHE HIS ASP ASN GLU GLU THR PHE LEU LYS LYS TYR LEU


                   545                    560                    575                        590
TAT GAA ATT GCC AGA AGA CAT CCT TAC TTT TAT GCC CCG GAA CTC CTT TTC TTT GCT AAA
TYR GLU ILE ALA ARG ARG HIS PRO TYR PHE TYR ALA PRO GLU LEU LEU PHE PHE ALA LYS


*FIGURE 4*

EP 0 198 745 B1

```
          605              620              635              650
AGG TAT AAA GCT GCT TTT ACA GAA TGT TGC CAA GCT.GCT GAT AAA GCA GCC TGC CTG TTG
ARG TYR LYS ALA ALA PHE THR GLU CYS CYS GLN ALA ALA ASP LYS ALA ALA CYS LEU LEU


          665              680              695              710
CCA AAG CTC GAT GAA CTT CGG GAT GAA GGG AAG GCT TCG TCT GCC AAA CAG AGA CTC AAG
PRO LYS LEU ASP GLU LEU ARG ASP GLU GLY LYS ALA SER SER ALA LYS GLN ARG LEU LYS


          725              740              755              770
TGT GCC AGT CTC CAA AAA TTT GCA GAA AGA GCT TTC AAA GCA TGG GCA GTA GCT CGC CTG
CYS ALA SER LEU GLN LYS PHE GLY GLU ARG ALA PHE LYS ALA TRP ALA VAL ALA ARG LEU


          785              800              815              830
AGC CAG AGA TTT CCC AAA GCT GAG TTT GCA GAA GTT TCC AAG TTA GTG ACA GAT CTT ACC
SER GLN ARG PHE PRO LYS ALA GLU PHE ALA GLU VAL SER LYS LEU VAL THR ASP LEU THR


          845              860              875              890
AAA GTC CAC ACG GAA TGC TGC CAT GGA GAT CTG CTT GAA TGT GCT GAT GAC AGG GCG GAC
LYS VAL HIS THR GLU CYS CYS HIS GLY ASP LEU LEU GLU CYS ALA ASP ASP ARG ALA ASP


          905              920              935              950
CTT GCC AAG TAT ATC TGT GAA AAT CAA GAT TCG ATC TCC AGT AAA CTG AAG GAA TGC TGT
LEU ALA LYS TYR ILE CYS GLU ASN GLN ASP SER ILE SER SER LYS LEU LYS GLU CYS CYS


          965              980              995              1010
GAA AAA CCT CTG TTG GAA AAA TCC CAC TGC ATT GCC GAA GTG GAA AAT GAT GAG ATG CCT
GLU LYS PRO LEU LEU GLU LYS SER HIS CYS ILE ALA GLU VAL GLU ASN ASP GLU MET PRO


          1025             1040             1055             1070
CCT GAC TTG CCT TCA TTA GCG GCT GAT TTT GTT GAA AGT AAC GAT GTT TGC AAA AAC TAT
ALA ASP LEU PRO SER LEU ALA ALA ASP PHE VAL GLU SER LYS ASP VAL CYS LYS ASN TYR


          1085             1100             1115             1130
GCT GAG GCA AAG GAT GTC TTC TTG GGC ATG TTT TTG TAT GAA TAT GCA AGA AGC CAT CCT
ALA GLU ALA LYS ASP VAL PHE LEU GLY MET PHE LEU TYR GLU TYR ALA ARG ARG HIS PRO
```

*FIGURE 1*

```
              1145              .        1160              1175              1190
GAT TAC TCT GTC GTA CTG CTG CTG AGA CTT GCC AAG ACA TAT GAA ACC ACT CTA GAG AAG
ASP TYR SER VAL VAL LEU LEU LEU ARG LEU ALA LYS THR TYR GLU THR THR LEU GLU LYS


              1205              1220              1235              1250
TGC TGT GCC GCT GCA GAT CCT CAT GAA TGC TAT GCC AAA GTG TTC GAT GAA TTT AAA CCT
CYS CYS ALA ALA ALA ASP PRO HIS GLU CYS TYR ALA LYS VAL PHE ASP GLU PHE LYS PRO


              1265              1280              1295              1310
CTT ATG GAA GAG CCT CAG AAT TTA ATC AAA CAA AAT TGT GAG CTT TTT GAG CAG CTT GGA
LEU MET GLU GLU PRO GLN ASN LEU ILE LYS GLN ASN CYS GLU LEU PHE GLU GLN LEU GLY


              1325              1340              1355              1370
GAG TAC AAA TTC CAG AAT GCG CTA TTA GTT CGT TAC ACC AAG AAA GTA CCC CAA GTG TCA
GLU TYR LYS PHE GLN ASN ALA LEU LEU VAL ARG TYR THR LYS LYS VAL PRO GLN VAL SER


              1385              1400              1415              1430
ACT CCA ACT CTT GTA GAG GTC TCA AGA AAC CTA GGA AAA GTG GGC AGC AAA TGT TGT AAA
THR PRO THR LEU VAL GLU VAL SER ARG ASN LEU GLY LYS VAL GLY SER LYS CYS CYS LYS


               1445              1460              1475              1490
CAT CCT GAA GCA AAA AGA ATG CCC TGT GCA GAA GAC TAT CTA TCC GTG GTC CTG AAC CAG
HIS PRO GLU ALA LYS ARG MET PRO CYS ALA GLU ASP TYR LEU SER VAL VAL LEU ASN GLN


              1505              1520              1535              1550
TTA TGT GTG TTG CAT GAG AAA ACG CCA GTA AGT GAC AGA GTC ACC AAA TGC TGC ACA GAA
LEU CYS VAL LEU HIS GLU LYS THR PRO VAL SER ASP ARG VAL THR LYS CYS CYS THR GLU


              1565              1580              1595              1610
TCC TTC GTG AAC AGG CCA CCA TGC TTT TCA GCT CTG GAA GTC GAT GAA ACA TAC GTT CCC
SER LEU VAL ASN ARG ARG PRO CYS PHE SER ALA LEU GLU VAL ASP GLU THR TYR VAL PRO


              1625              1640              1655              1670
AAA GAC TTT AAT GCT GAA ACA TTC ACC TTC CAT GCA GAT ATA TGC ACA CTT TCT GAG AAG
LYS GLU PHE ASN ALA GLU THR PHE THR PHE HIS ALA ASP ILE CYS THR LEU SER GLU LYS
```

*FIGURE 4*

1685      1700      1715      1730

GAG AGA CAA ATC AAG AAA CAA ACT GCA CTT GTT GAG CTT GTG AAA CAC AAG CCC AAG GCA

GLU ARG GLN ILE LYS LYS GLN THR ALA LEU VAL GLU LEU VAL LYS HIS LYS PRO LYS ALA


1745      1760      1775      1790

ACA AAA GAG CAA CTG AAA GCT GTT ATG GAT GAT TTC GCA GCT TTT GTA GAG AAG TGC TGC

THR LYS GLU GLN LEU LYS ALA VAL MET ASP ASP PHE ALA ALA PHE VAL GLU LYS CYS CYS


1805      1820      1835      1850

AAG GCT GAC GAT AAG GAA ACC TGC TTT GCC GAG GAG GGT AAA AAA CTT GTT GCT GCA AGT

LYS ALA ASP ASP LYS GLU THR CYS PHE ALA GLU GLU GLY LYS LYS LEU VAL ALA ALA SER


1865      1880      1895

CAA GCT GCC TTA GGC TTA TAA CAT CAC ATT TAA AAG CAT CTC AGC CTA CCA

GLN ALA ALA LEU GLY LEU  (585-STOP)


*FIGURE 4*

```
1  2  3
```

#1--- 0,5 microgrammes de HSA naturelle (SIGMA)
#2--- 1 microgramme de HSA naturelle (SIGMA)
#3--- Protéines insolubles, contenant l'HSA recombinante,
      correspondant à 100 microlitres de culture bactérienne
      à densité optique de 4 $(A_{610})$

Figure 5

# DETECTION DE LA RENATURATION
# DE LA SERUM-ALBUMINE HUMAINE PAR DOSAGE
# ELISA : PRINCIPE DE LA METHODE

Sérum-albumine humaine native

Anticorps monoclonal anti-conformation native

cupule de plaque d'immuno-titration

**Excès de sérum-albumine native dans la préincubation** ➡ **PAS DE SIGNAL**

sérum-albumine dénaturée + autres molécules

Anticorps anti-IgG de souris marqué à la phosphatase alcaline

**Préincubation en présence de sérum-albumine dénaturée** ➡ **SIGNAL**

Figure 6

Comparaison de la sérum-albumine humaine
authentique et de la sérum-albumine humaine
préparé par le procédé de préparation microbiologique

Gel de polyacrylamide natif 10%

A- sérum-albumine humaine (Sigma)

B- sérum- albumine humaine recombinante
    après renaturation

Figure 7